# EUROPEAN PATENT SPECIFICATION

(11) **EP 3 871 665 B1**
(45) Date of publication and mention of the grant of the patent: **24.12.2025**
(21) Application number: 20159945.3
(22) Date of filing: 28.02.2020
(51) Int. Cl.: A61K 31/165, A61K 31/4245, A61P 35/00, G01N 33/50

(54) **INHIBITORS OF RNA-BINDING PROTEINS AS ANTI-CANCER DRUGS**
INHIBITOREN VON RNA-BINDENDEN PROTEINEN ALS ANTIKREBSMITTEL
INHIBITEURS DE PROTÉINES DE LIAISON ARN EN TANT QUE MÉDICAMENTS ANTICANCÉREUX

(43) Date of publication of application: 01.09.2021
(73) Proprietor: Martin-Luther-Universität Halle-Wittenberg, 06108 Halle (Saale) (DE); Cambridge Enterprise Limited, Cambridge Cambridgeshire CB2 1TN (GB)
(72) Inventor: Hüttelmaier, Stefan, 06114 Halle (DE); Borges Bertoldo, Jean, 06114 Halle (DE); Bernardes, Gonçalo, Cambrige Cambridgeshire CB2 1EW (GB); Müller, Simon, 06110 Halle (DE)
(74) Representative: Maikowski & Ninnemann Patentanwälte Partnerschaft mbB

(56) References cited:
- LILY MAHAPATRA ET AL: "A Novel IMP1 Inhibitor, BTYNB, Targets c-Myc and Inhibits Melanoma and Ovarian Cancer Cell Proliferation", TRANSLATIONAL ONCOLOGY, vol. 10, no. 5, 1 October 2017 (2017-10-01), United States, pages 818 - 827, XP055718562, ISSN: 1936-5233, DOI: 10.1016/j.tranon.2017.07.008
- ZHOU X ET AL: "miR-625 suppresses tumour migration and invasion by targeting IGF2BP1 in hepatocellular carcinoma", ONCOGENE, NATURE PUBLISHING GROUP UK, LONDON, vol. 34, no. 8, 17 March 2014 (2014-03-17), pages 965 - 977, XP036973302, ISSN: 0950-9232, [retrieved on 20140317], DOI: 10.1038/ONC.2014.35
- GHOSHAL ARCHITA ET AL: "Extracellular vesicle-dependent effect of RNA-binding protein IGF2BP1 on melanoma metastasis", ONCOGENE, NATURE PUBLISHING GROUP UK, LONDON, vol. 38, no. 21, 1 April 2019 (2019-04-01), pages 4182 - 4196, XP036851853, ISSN: 0950-9232, [retrieved on 20190401], DOI: 10.1038/S41388-019-0797-3
- PHILIP R. LINDSTEDT ET AL: "Enhancement of the Anti-Aggregation Activity of a Molecular Chaperone Using a Rationally Designed Post-Translational Modification", ACS CENTRAL SCIENCE, vol. 5, no. 8, 28 August 2019 (2019-08-28), pages 1417 - 1424, XP055718159, ISSN: 2374-7943, DOI: 10.1021/acscentsci.9b00467

## Description

### Field of the invention

The invention relates to anti-proliferative compounds or a pharmaceutical composition comprising said anti-proliferative compounds for use in the prevention or treatment of cancer, tumor metastasis or tumor recurrence in a subject, wherein said anti-proliferative compound binds covalently to non-catalytic cysteine residues of an IGF2 mRNA-binding protein, thereby inhibiting the proliferative activity of said IGF2 mRNA-binding protein. The invention further relates to methods for identifying functionally relevant non-catalytic cysteine residues in RNA-binding proteins and for identifying anti-proliferative compounds, which bind chemo- and regio-selectively to functionally relevant non-catalytic cysteine residues of a RNA-binding protein. The invention is defined by the appended claims.

### Background of the invention

### Targeting cysteine residues in cancer-promoting RNA-binding proteins

RNA-binding proteins (RBPs) are critical players in cancer progression and their expression correlates directly with poor patient prognosis. Due to their role in cancer, RBPs have attracted attention in drug development over the years (see US6004749A). However, targeting RBPs has proven to be difficult due to the mostly non-enzymatic nature of RNA-protein association. Thus, specific RNA-protein association inhibitors are not so frequently discovered or designed, with only a few examples described. A promising way to overcome this shortcoming is targeting key cysteine residues in these proteins, since there is evidence that they play essential roles in protein ligand-binding and cellular function of cancer-promoting proteins. In fact, targeting cysteine residues in disease relevant proteins has allowed the generation of potent FDA-approved drugs such as ibrutinib. This drug is able to target the Cys481 residue in the ATP binding domain of the Bruton's tyrosine kinase and is successfully used to treat B-cell malignancies (see US9730938B2). Several inhibitors have since been designed to target specific cysteines showing that this residue is indeed a critical target for drug design. Nevertheless, in non-catalytic proteins (i.e RBPs), the targeting of cysteine residues is not as intuitive as it is in enzymes. Accordingly, targeting cysteine residues in RBPs has not been considered to develop drugs impairing their function in cancer cells so far. This leaves the question of which cysteine, in the case of multiple-Cys-containing RBPs, is critical for protein activity and therefore an interesting target for covalent inhibition. To address this challenge, chemical approaches using compounds to specifically modify cysteines arise as promising methodologies. Several compounds were designed over the years to fulfil this role including, the 2,5-dibromohexanediamide (DBHDA; disclosed in WO2009103941A1). This molecule is able to target cysteine residues site-selectively, converting these to dehydroalanine (Cys-to-Dha approach). DBHDA has been used to facilitate the incorporation of non-natural posttranslational modifications in proteins with the aim to produce protein-drug conjugates and to expand protein function.

The current cysteine modification methodologies have been exclusively applied to proteins containing a single Cys residue, and therefore uniquely used for protein functionalization in order to produce antibody-drug conjugates. This includes the Cys-to-Dha approach (see WO2009103941A1). No mention in the literature is found about using a Dha-based cysteine modification approach to multiple-Cys-containing proteins in order to reveal functionally relevant Cys residues, except in Bertoldo et al., 2017, 2018. However, Bertoldo et al., 2017, 2018 worked on enzymes, namely bacterial phosphatases, which are catalytic proteins.

### IGF2BP1 is a bona fide target in cancer therapy

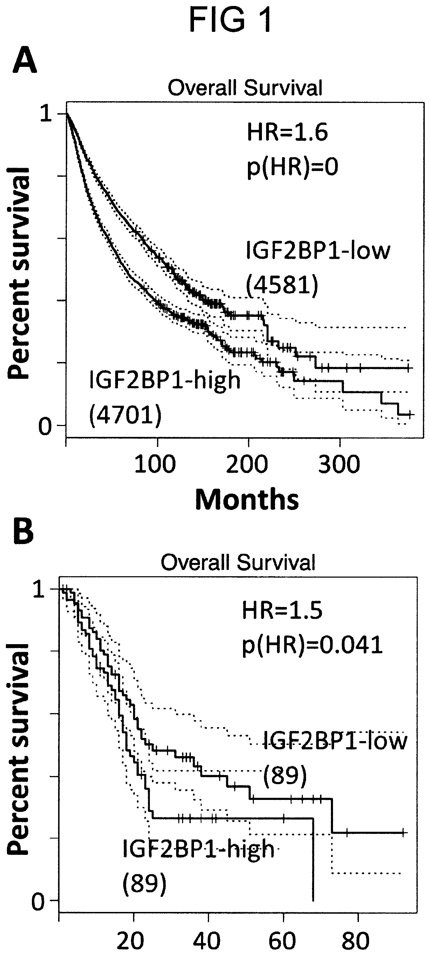

The IGF2 mRNA binding protein family comprises three non-catalytic, conserved RBPs (Bell et al., 2013). Two members of the family, IGF2BP1 and 3, are *bona fide* oncofetal proteins mainly expressed during embryogenesis, absent in the vast majority of adult tissues, and *de novo* synthesized in various malignancies (Bell et al., 2013). Despite distinct expression patterns, all three IGF2BPs were considered to play essential roles in normal stem and progenitor cells, as well as cancer stem cells (Degrauwe et al., 2016). In agreement, IGF2BPs were shown to promote tumor cell proliferation, self-renewal, migration, invasion, tumor growth and metastasis in experimental tumor models, most prominently shown for IGF2BP1 (Gutschner et al., 2014; Muller et al., 2018). The main role of IGF2BP1 in cancer cells is the stabilization of target mRNAs resulting in the upregulated expression of oncogenes or oncogenic factors like MYC family proteins, LIN28B and MKI67 (Ki- 67) (Bell et al., 2015; Busch et al., 2016; Gutschner et al., 2014; Kobel et al., 2007). Among IGF2BPs, IGF2BP1 shows the most conserved oncogenic potential in solid cancer models (Muller et al., 2018). Consistently, the protein is substantially upregulated in various solid cancers, e.g. epithelial ovarian cancer, hepatocellular carcinoma as well as neuroblastoma (Bell et al., 2015; Gutschner et al., 2014; Kobel et al., 2007). Notably, enhanced expression of IGF2BP1 is most prominently observed in progressed stages of these malignancies and associated with adverse patient prognosis (Bell et al., 2015; Kobel et al., 2007; Muller et al., 2018). The investigation of 33 cancer transcriptomes (via GEPIA2; link: http://gepia2.cancerpku.cn ) revealed that IGF2BP1 mRNA expression is substantially increased in the vast majority of solid cancers (data not shown). In most of these cancers IGF2BP1 abundance is associated with and adverse patient prognosis, indicated here for all 33 cancers analyzed via GEPIA2 and pancreatic ductal adenocarcinoma, one of the most lethal cancers with a five year survival of less than 9% (Figure 1A, B). These findings suggest IGF2BP1 as a *bona fide* target for cancer therapy.

Despite bulk evidence for a pro-oncogenic role of IGF2BP1, only one small molecule inhibitor of IGF2BP1, termed BTYNB (Mahapatra et al., 2017), has been reported to date. This small molecule inhibitor was discovered by high-throughput screening of small molecules interfering with the association of recombinant IGF2BP1 and a fragment of the MYC RNA. In IGF2BP1-expressing cancer-derived tumor cells, BTYNB impaired cell vitality, whereas this was substantially less pronounced in cells expressing IGF2BP1 at low levels. However, a potential potency of BTYNB in impairing tumor growth *in vivo* has not been investigated to date. Moreover, how BTYNB impairs IGF2BP1-RNA association remains largely elusive. Nonetheless, these findings suggest that the inhibition of pro-oncogenic roles of IGF2BP1 in cancer cells is feasible. This supports the notion that IGF2BP1 is a *bona fide* candidate target for pursuing novel avenues in developing small molecule inhibitors for cancer therapy.

Moreover, ZHOU X ET AL, ONCOGENE, NATURE PUBLISHING GROUP UK, LONDON, vol. 34, no. 8 (2014), pages 965-977 discloses that mi R-625 suppresses tumour migration and invasion by targeting IGF2BP1 in hepatocellular carcinoma. GHOSHAL ARCH IT A ET AL, ONCOGENE, NATURE PUBLISHING GROUP UK, LONDON, vol. 38, no. 21 (2019), pages 4182-4196, report on the extracellular vesicle-dependent effect of RNA-binding protein IGF2BP1 on melanoma metastasis.

### Summary of the invention

Taken together, non-catalytic proteins play a crucial role in cancer development, tumor growth and metastasis. Accordingly, there is a great need for agents, which are able to interfere with the interaction between non-catalytic proteins and their binding partners, resulting in new specific and effective cancer treatments. This problem is solved by the present invention.

Accordingly, the invention provides anti-proliferative compounds or a pharmaceutical composition comprising said anti-proliferative compounds for use in the prevention or treatment of cancer, tumor metastasis or tumor recurrence in a subject, wherein said anti-proliferative compound binds chemo- and regio-selectively to functionally relevant non-catalytic cysteine residues of a RNA-binding protein, said RNA-binding protein comprising multiple cysteine residues, wherein said anti-prolifrative compound is selected from *(E)-N-*(2-((7-nitrobenzo[c][1,2,5]oxadiazol-4-yl)amino)ethyl)-4-oxo-4-phenylbut-2-enamide and (E)-N-ethyl-4-oxo-4-phenylbut-2-enamide and wherein said RNA-binding protein is a IGF2 mRNA-binding protein.

The invention further provides a method for identifying targetable cysteine residues in RNA-binding proteins, which are mostly non-catalytic proteins and which are selected from IGF2 mRNA-binding proteins. This method is based, at least in part, on the surprising finding that the compound 2,5-dibromohexanediamide is also able to site-selectively target non-catalytic cysteines in RBPs according to Cys intrinsic reactivity. The specific Cys' hyper-reactivity leads to its preferential modification by the compound and reveal, its otherwise unknown, critical role in the RBPs activity. Accordingly, in a first aspect the invention provides a method that allows the identification of critical cysteines residues, linked to the RBPs ability to induce aggressive cancer phenotypes, thus, providing a target for a drug design platform in cancer relevant RBPs. Currently there are no specific platforms aiming at identifying and modifying cysteine residues in RBPs with the purpose to reveal targetable sites for a drug discovery program in cancer. On the other hand, there are several methodologies that allow site-selective modification of cysteine residues in proteins. Other techniques include non-targeted, unbiased compound screening assays to discover synthetic small molecules or natural compounds able to inhibit the RNA association of RBPs.

The method of the invention enables the fast and reliable identification of targetable cysteine residues in RBPs critical for their association with and regulation of "oncogenic RNAs" (coding and noncoding RNAs). The identified residues then become promising targets for a drug design program in cancer. The final goal is achieved by developing specific cysteine-directed covalent inhibitors aimed at the identified residues in the protein target. This platform is particularly suitable for non-catalytic proteins where finding a motif or binding pocket to design an inhibitor is considerably difficult. It is also very well suitable in protein targets with multiple cysteine residues and it allows the fast identification of worth-investigating residues.

### Brief description of the figures

**Figure 1** shows that IGF2BP1 expression is associated with adverse prognosis in cancer. **(A)** Survival analysis (median cutoff) of IGF2BP1 mRNA expression in 33 cancers (9282 cancer samples) via GEPIA2. **(B)** Survival analysis (median cutoff) in pancreatic ductal adenocarcinoma (PAAD; 178 samples). Statistical significance was analyzed by logrank test. HR, Hazard ratio (HR).

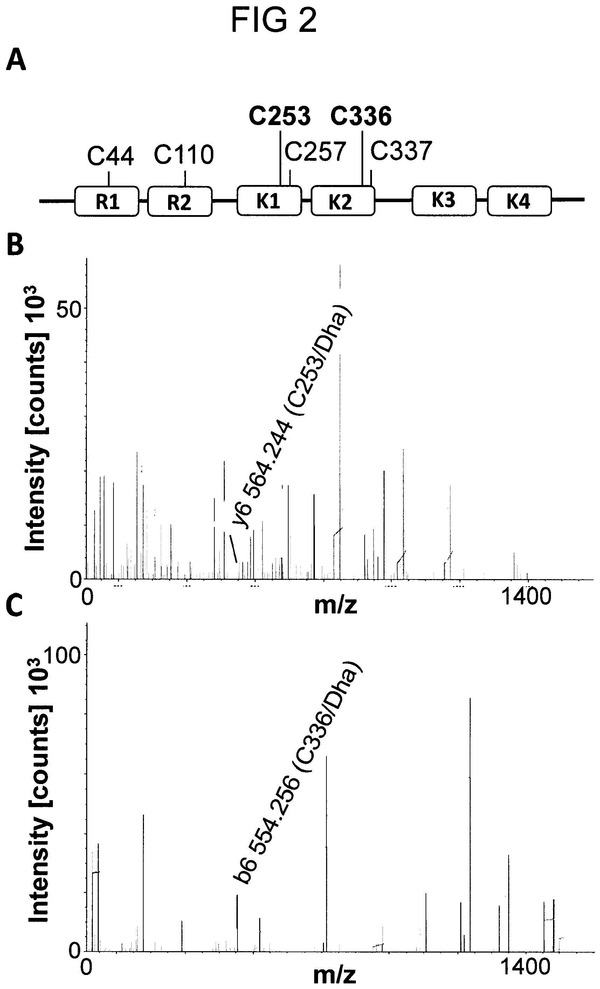

**Figure 2** shows the identification of cysteine residues converted to DHA by DBHDA. **(A)** Schematic of the human IGF2BP1 protein with domains (R: RRM, RNA recognition motif; K: KH, HNRNPK-homology domain) and cysteines (C, numbers indicate position in primary sequence). Modified cysteines are highlighted in bold and upper position. **(B, C)** MS/MS spectra of Dha-modified peptides with (A) Peptide AISVHSTPEGCSSACK (SEQ ID NO: 4) containing the Cys-to-Dha conversion at Cys253 (modification achieved using 200 eq of 1) and (B) Peptide GAIENCCRAEQEIMK (SEQ ID NO: 5) containing the Cys-to-Dha conversion at Cys336 (achieved using 300 eq of 1).

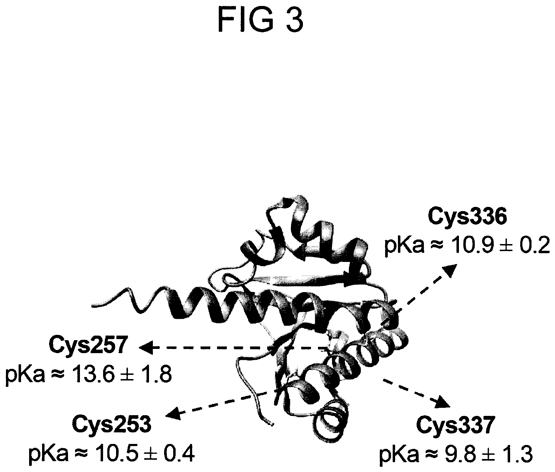

**Figure 3** shows the predicted pKa values of cysteine residues in IGF2BP1 KH domains 1 and 2 using 1 uS molecular dynamic simulations (PDB 6QEY).

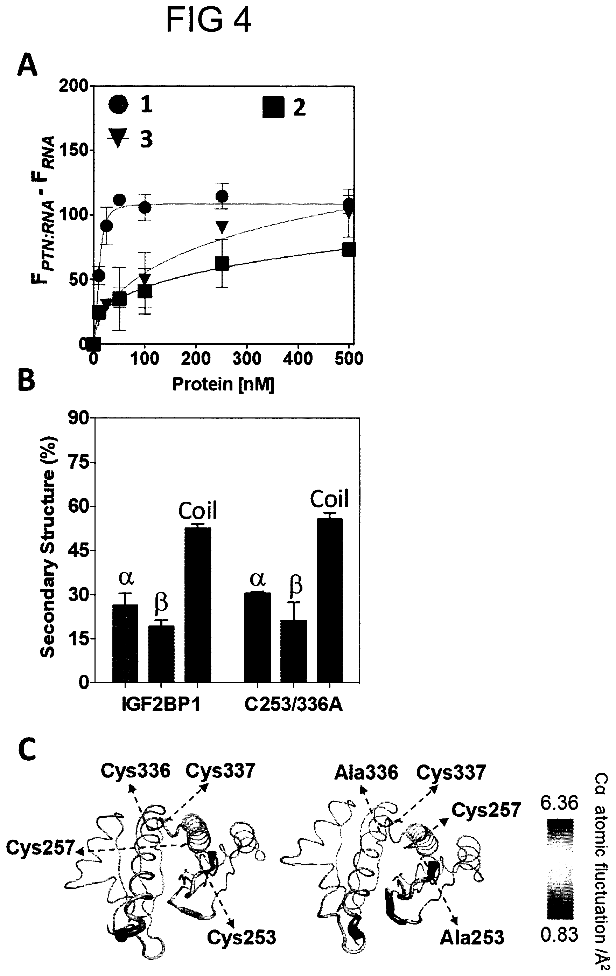

**Figure 4** shows the structural and functional analyses of Cys253 and Cys336 impact on IGF2BP1 RNA binding activity. **(A)** Fluorescent (de)quenching assay using a 5'FAM labeled oligo RNA derived from the c-Myc mRNA coding region. 100 nM of oligo RNA was incubated in the absence or in the presence of increasing concentrations of IGF2BP1-KH1-4 wild type (1), IGF2BP1-KH1-4-C253/336A (2) or DBHDA-treated IGF2BP1-KH1-4 (3). Fluorescent emission of protein/RNA complex was subtracted from fluorescent emission of oligo RNA alone. **(B)** Secondary structure content quantification (α: a-helix; β: ß-strand; coil: random coil) obtained from CD spectra of IGF2BP1 and C253/336A mutant in the absence of RNA. Secondary structure estimation was obtained by plotting raw CD data against a CD data bank performed with the algorithm K2D3. **(C)** 1 µS molecular dynamic simulations performed with crystal structure of IGF2BP1 KH1-2 (PDB 6QEY). Right: Local atomic fluctuations around cysteine residues. Left: Atomic fluctuations derived from Cys-to-Ala mutation of Cys253 and Cys336.

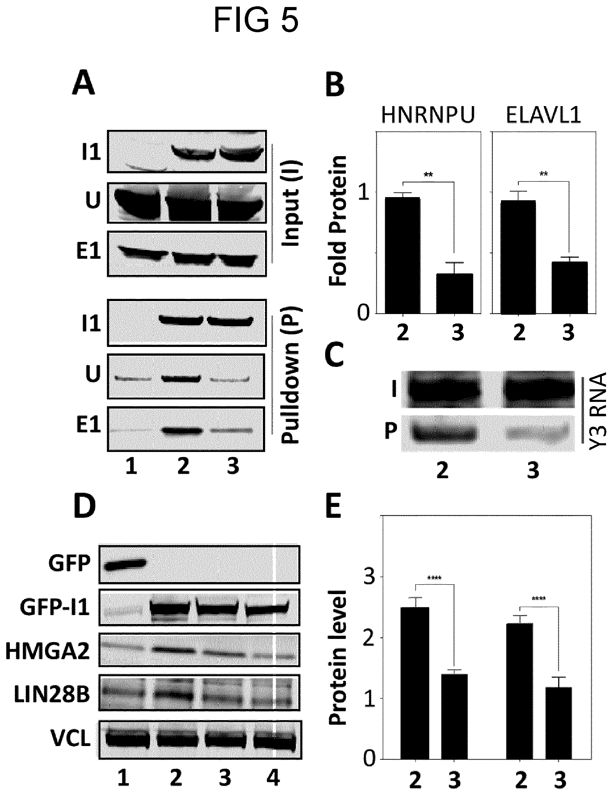

**Figure 5** shows that Cys253/336 are essential for proper IGF2BP1 function in cancer cells. **(A-C)** RNA-dependent protein-protein association assay. Co-purification of proteins, hnRNPU (U), ELAVL1 (E1) in (A, B) and the non-coding Y3 RNA in (C) from intracellular RNP complexes upon pulldown of transiently expressed SBP-tagged wild type IGF2BP1 (I1, 2) and IGF2BP1-C253/336A (C253/336A, 3) from transfected ES-2 cells. EV (1) indicates mock-transfected controls. Representative Western (A) and Northern (C) blots are shown. P, pulldown; I, input. Amounts of indicated protein copurified (B) with IGF2BP1-C253/336A were determined in three independent analyses relative to copurification with wild type IGF2BP1. **(D,E)** Protein expression level of IGF2BP1 targets (HMGA2 and LIN28B) analyzed in ES-2 IGF2BP1-KO cells stably re-expressing GFP (GFP, 1), wild type GFP-IGF2BP1 (GFP-I1, 2), the Cys253/336Ala mutant (GFP-C253/336A, 3) or RNA-binding deficient IGF2BP1 (GFP-KHmut, 4). A representative Western blot analysis is shown in (C). Protein levels (D) relative to GFP-only controls were analyzed by Western blotting and quantified in three independent experiments using VCL as an internal normalization control. Statistical significance was calculated by unpaired t-test with Welch's correction. (**) p < 0.01 and (***) p < 0.001.

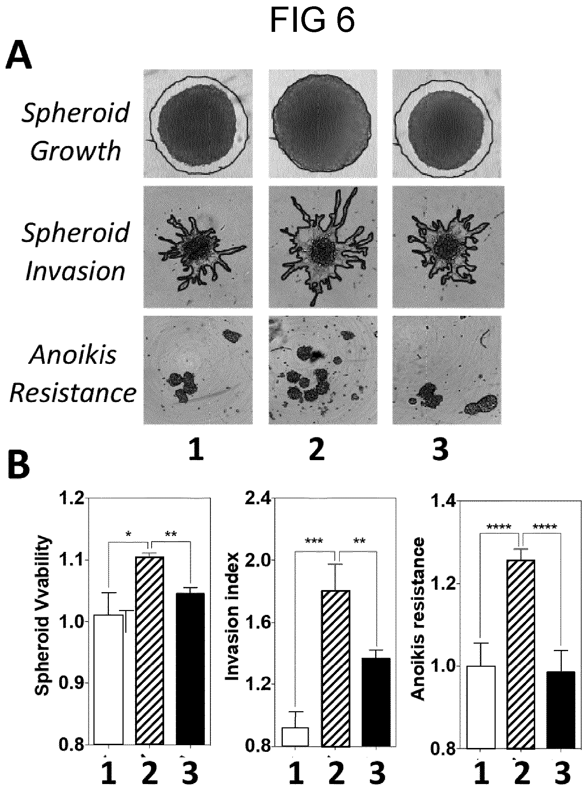

**Figure 6** shows that Cys253/336 are essential for IGF2BP1-induced tumor cell properties. **(A)** Representative images of tumor cell spheroids (top panel), tumor cell invasion in matrigel (middle panel), and spheroid growth in anoikis resistance assays (lower panel) observed for ES-2 IGF2BP1-KO cells re-expressing GFP (1), wild type GFP-IGF2BP1 (2) or GFP- IGF2BP1-C253/336A (3). **(B)** Quantification of 2 independent experiments (n=9) shown in (A): spheroid viability (left panel), invasion index (middle panel) and anoikis resistance (right panel). Statistical significance was calculated by unpaired t-test as indicated. (*) p < 0.05, (**) p < 0.01, (***) p < 0.001, (****) p < 0.0001.

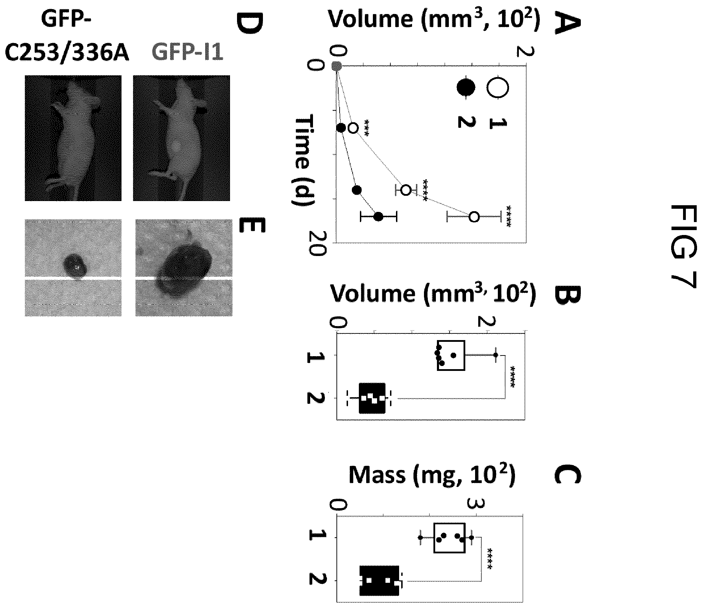

**Figure 7** shows that Cys253/336 of IGF2BP1 are essential for the tumor growth-promoting role of IGF2BP1 in experimental tumor models. Tumor formation was determined for ES-2 IGF2BP1-KO cells re-expressing wild type IGF2BP1 (GFP-I1, 1) or GFP-IGF2BP1-C253/336A (GFP-C253/336A, 2). The tumor volume was measured at indicated time points post-injection by a caliper, **(A).** The final volume **(B)** and weight **(C)** of removed tumors were determined and are shown by boxplots. Representative images of macroscopic tumors acquired by non-invasive infrared imaging in mice **(D)** and excised tumors 17d post-injection **(E).** Six mice were analyzed per condition. Error bars indicate standard deviation. Statistical significance was calculated by unpaired t test with Welch's correction or ordinary one-way ANOVA with Tukey's multiple comparison test. (***) p < 0.001.

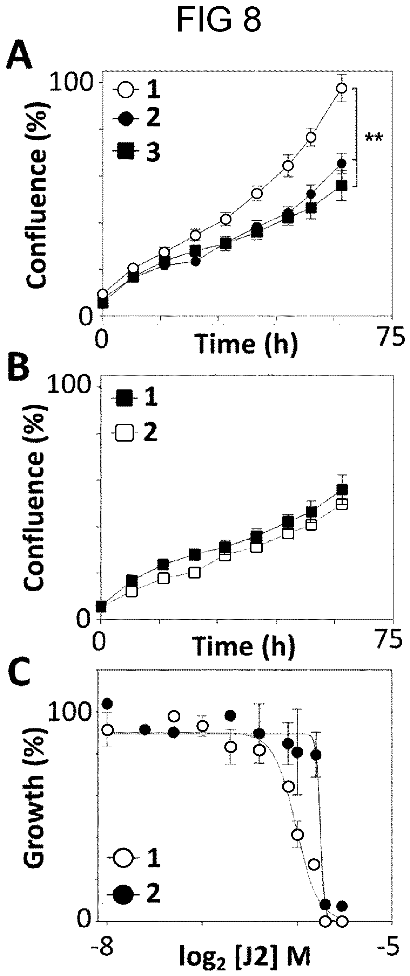

**Figure 8** shows that J2 impairs tumor cell proliferation in a IGF2BP1-Cys253/336-dependent manner. 2000 cells either from ES-2 IGF2BP1-KO cells re-expressing GFP-IGF2BP1 or GFP-IGF2BP1_{C253/336A} were seeded in a 96 well plate. The cells were allowed to sit overnight and in the next day were incubated with indicated fixed or increasing concentrations of J2. Cell growth was measured by an IncuCyte S3 as means of confluence. **(A)** Comparison between growth curves of GFP-IGF2BP1 cells treated with DMSO (1), or with 1µM of J2 and GFP-IGF2BP1_{C253/336A} (3) cells. **(B)** Comparison of growth curves between GFP-IGF2BP1_{C253/3364} cells and GFP-IGF2BP1_{C253/336A} treated with DMSO (1) or with 1µM of J2 (2). **(C)** IC50 determination of J2 potency against GFP-IGF2BP1 (1) and GFP-IGF2BP1_{C253/336A} (2) cells. Confluence of DMSO-treated cells at 72h was assumed as 100% growth. Error bars indicate standard deviation of three analyses. Statistical significance was calculated by One-way ANOVA with Holm-Sidak's multiple comparison test. *(**) p < 0.01.*

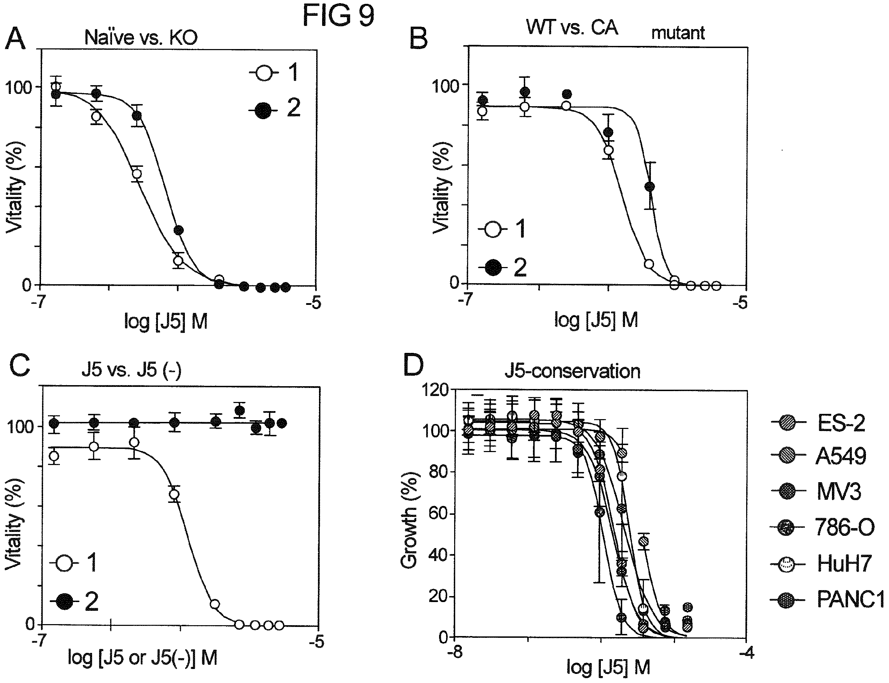

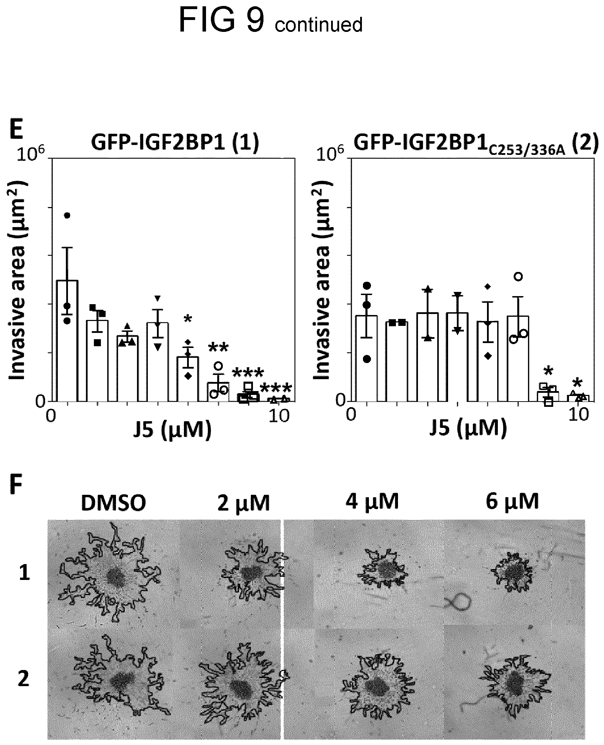

**Figure 9** shows that J5 is a potent IGF2BP1-targeting anti-cancer small molecule. **(A)** Inhibition profile of J5 against naive parental ES-2 cells (1) compared to ES-2 IGF2BP1-KO (2) cells. **(B)** Inhibition profile of J5 against ES-2 IGF2BP1-KO cells re-expressing the wild type GFP-IGF2BP1 (1) and the GFP-IGF2BP1_{C253/336A} mutant (2). **(C)** Inhibition profile of J5 (1) and inactive (non-cysteine reactive; 2) J5 (-) against ES-2 IGF2BP1-KO re-expressing the wild type IGF2BP1. **(D)** Inhibition profile of J5 against a panel of indicated cancer cell lines. 2000 cells were seeded in each well and left to adhere for 24 h. The next day, increasing concentrations of J5 were added to the wells. Cell viability was measured at 72h post-incubation with CellTiter Glo^{®} (A-C) and cell proliferation with IncuCyte S3 (D) as means of cell confluence (growth). Cell viability and proliferation of DMSO-treated cells was considered as 100%. Data is represented as mean ± SEM (n=4 for each condition). **(E)** J5 invasion inhibition of ES-2 IGF2BP1-KO cells re-expressing the wild-type IGF2BP1 (GFP-IGF2BP1; left panel) and the mutant C253/336A (GFP-IGF2BP1_{C253/336A}; right panel). 1000 cells were seeded in a 96-well concave ultra-low attachment plate and let to grow for 24 h. In the next day, 50 µL of Matrigel Matrix^{®} was added to pre-formed spheres followed by increasing concentrations of J5. The invasion was measured 24h post-treatment using IncuCyte Largest invasive area module. DMSO-treated cells were used for comparisons. Data is represented as mean ± SD (n=4 for each condition). Statistical significance was calculated by One-way ANOVA with Holm-Sidak's multiple comparison test. (*) p < 0.05, (**) p < 0.01, (***) p < 0.001. **(F)** Representative images of spheroid invasion observed in ES-2 IGF2BP1-KO cells re-expressing GFP-IGF2BP1 or GFP-IGF2BP1_{C253/336A} in the presence of increasing J5 concentrations.

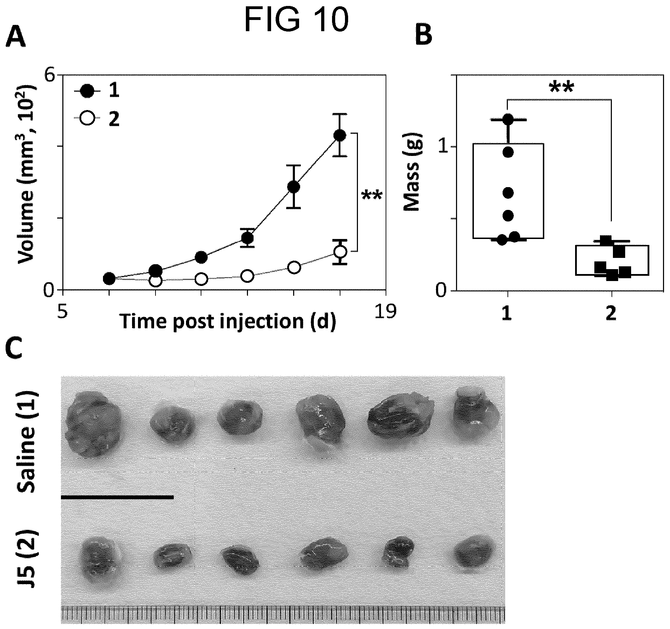

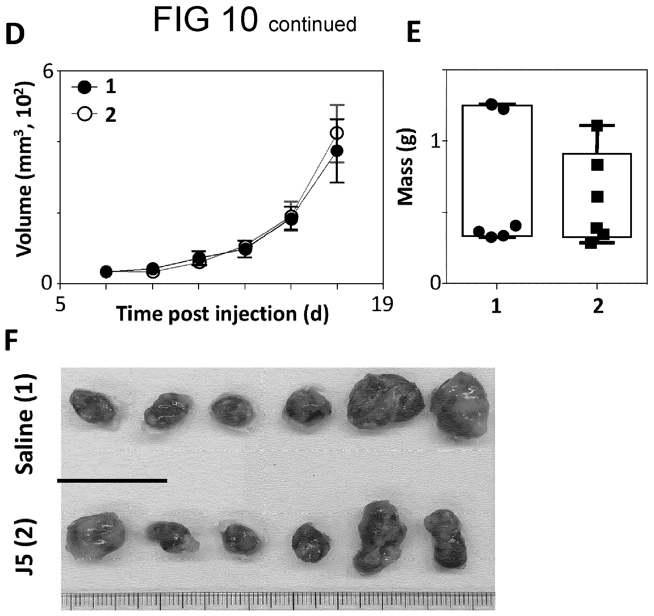

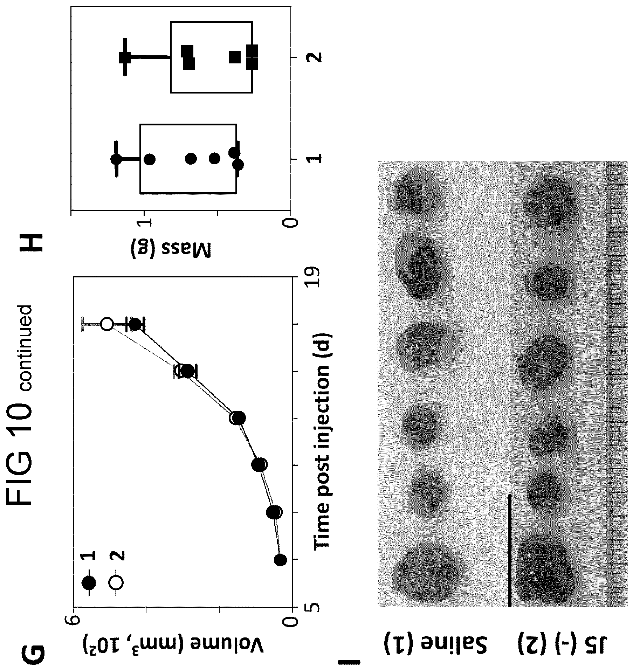

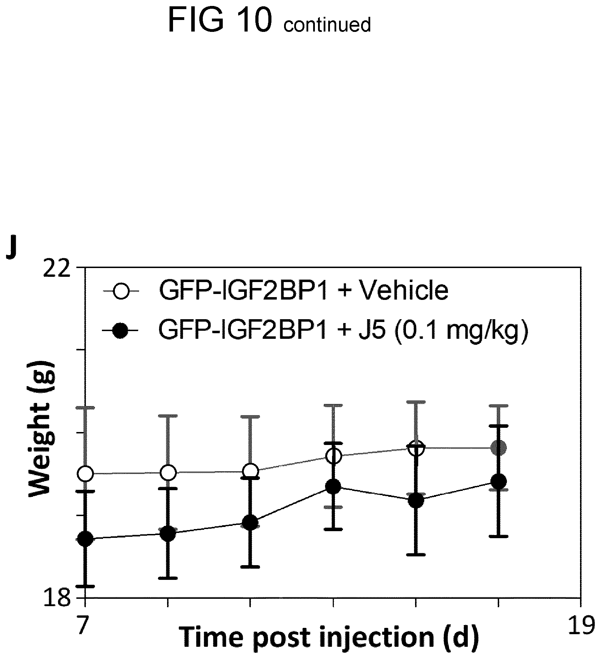

**Figure 10** shows that J5 is a potent IGF2BP1-targeting anti-tumor drug. Tumor volume **(A)** and tumor mass **(B)** of Xenografts bearing ES-2 IGF2BP1-KO cells re-expressing the wild type IGF2BP1 (GFP-IGF2BP1) treated with saline (1) or J5 (2). **(C)** Images of primary tumors removed by surgery when the saline-treated tumors reached the termination criterion. Tumor volume **(D)** and tumor mass **(E)** of Xenografts bearing ES-2 IGF2BP1-KO cells re-expressing the mutant C253/336A (GFP-IGF2BP1_{C253/336A}) treated with saline (1) or J5 (2). **(F)** Images of primary tumors removed by surgery when the saline-treated tumors reached the termination criterion. Tumor volume **(G)** and tumor mass **(H)** of Xenografts bearing ES-2 IGF2BP1-KO cells re-expressing the wild type IGF2BP1 (GFP-IGF2BP1) treated with saline (1) or J5 (-) (2). **(I)** Images of primary tumors removed by surgery when the saline-treated tumors reached the termination criterion. The tumor volume was measured at indicated time points post-injection by a caliper. Data represent the mean ± SD (n = 6). Statistical significance was calculated by unpaired t-test with Man Whitney correction or ordinary one-way ANOVA with Tukey's multiple comparison test. (**) p < 0.01. **(J)** Mouse weight measured upon injection of ES-2 IGF2BP1-KO cells re-expressing GFP-IGF2BP1 over the course of treatment with either saline or J5. All compounds were injected at 0.1 mg/kg body weight.

### Detailed description of the invention

The invention provides anti-proliferative compounds or a pharmaceutical composition comprising said anti-proliferative compounds for use in the prevention or treatment of cancer, tumor metastasis or tumor recurrence in a subject, wherein said anti-proliferative compound binds chemo- and regio-selectively to non-catalytic cysteine residues, preferably to functionally relevant non-catalytic cysteine residues of a RNA-binding protein, said RNA-binding protein comprising multiple cysteine residues, wherein said anti-prolifrative compound is selected from *(E)*-*N*-(2-((7-nitrobenzo[c][1,2,5]oxadiazol-4-yl)amino)ethyl)-4-oxo-4-phenylbut-2-enamide and (E)-N-ethyl-4-oxo-4-phenylbut-2-enamide and wherein said RNA-binding protein is a IGF2 mRNA-binding protein.

"RNA-binding proteins" (RBPs) are proteins that bind to the double or single stranded RNA in cells and participate in forming ribonucleoprotein complexes. RBPs contain various structural motifs, such as RNA recognition motif (RRM), dsRNA binding domain, zinc finger and others. They are cytoplasmic and nuclear proteins. RBPs have crucial roles in various cellular processes such as: cellular function, transport and localization. They especially play a major role in post-transcriptional control of gene expression by modulating RNA fate and function, such as: splicing, polyadenylation, RNA turnover control, mRNA localization and translation. Eukaryotic cells encode diverse RBPs with unique RNA-binding activity and protein-protein interaction.

The term "RNA-binding protein" or "RBP" as used herein refers to RBPs comprising, preferably, multiple cysteine residues, more preferably at least one non-catalytic cysteine residue, which is functionally relevant. The RBP according to the invention is a protein selected from the insulin-like growth factor 2 (IGF2) mRNA binding protein family, wherein said family comprises three non-catalytic, conserved RBPs (Bell et al., 2013). The IGF2RBP is preferably selected from insulin-like growth factor 2 mRNA binding protein 1 (IGF2BP1 (SEQ ID NO: 1), 2 (IGF2BP2 (SEQ ID NO: 2 ) and 3 (IGF2BP3 (SEQ ID NO: 3). Most preferably, the IGF2RBP is insulin-like growth factor 2 mRNA binding protein 1 (IGF2BP1). It has been found surprisingly that IGF2BP1 comprises two non-catalytic cysteine residues at positions 253 and 336, when referring to the polypeptide of SEQ ID NO: 1, wherein mutating these two cysteine residues result in the inhibition of the pathological proliferative activity of IGF2BP1.

"Functionally relevant" in the context of the invention refers to cysteine residues, preferably non-catalytic cysteine residues, in a RBP, such as IGF2RBP, that mediate the binding of the RBP with a RNA. Inhibition of RBP-RNA association via modification of these functionally relevant cysteine residues leads to the prevention or treatment of pathological conditions, such as proliferative or pro-metastatic tumor cell potential. The anti-proliferative compounds of the invention inhibit the binding of the RBPs to their target mRNAs.

"Targetable cysteine residues" are cysteine residues that can be reached by the anti-proliferative compounds of the invention and which can covalently bind an anti-proliferative compound of the invention. Targetable cysteine residues are different from non-targetable cysteine residues. Non-targetable cysteine residues are not accessible for the anti-proliferative compounds of the invention, e.g. because of steric hindrance or because a covalent binding of an anti-proliferative compound of the invention is not possible for other reasons.

The "anti-proliferative compound" as used herein is a compound selected from *(E)-N-(2-((7-*nitrobenzo[c][1,2,5]oxadiazol-4-yl)amino)ethyl)-4-oxo-4-phenylbut-2-enamide and *(E)-N-*ethyl-4-oxo-4-phenylbut-2-enamide, including pharmaceutically acceptable salt, solvate or polymorph thereof, including all tautomers and stereoisomers thereof. *(E)-N-*ethyl-4-oxo-4-phenylbut-2-enamide and its fluorescent derivative the compound *(E)-N-(2-((7-*nitrobenzo[c][1,2,5]oxadiazol-4-yl)amino)ethyl)-4-oxo-4-phenylbut-2-enamide are known in the art to covalently modify cysteine residues in a highly site-specific manner (Bernardim et al., 2016). Previously, these compounds were used to reveal a functionally relevant cysteine in a chaperone (Lindstedt et al., 2019). An anti-proliferative activity of these compounds has not been reported so far.
*(E)-N-*(2-((7-nitrobenzo[c][1,2,5]oxadiazol-4-yl)amino)ethyl)-4-oxo-4-phenylbut-2-enamide has the structural formula: 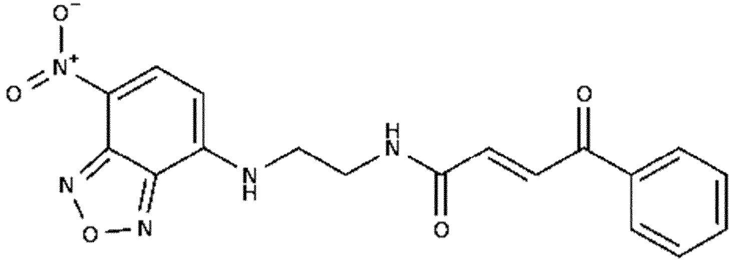
*(E)-N*-ethyl-4-oxo-4-phenylbut-2-enamide has the structural formula:

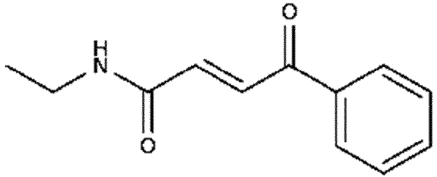

The anti-proliferative compound of the invention preferably binds to at least one non-catalytic cysteine residue of an RBP and thereby inhibits RBP activity. When the RBP is IGF2BP1, the anti-proliferative compound of the invention preferably binds to at least one of the two non-catalytic cysteine residues at positions 253 and 336 (when referring to the polypeptide of SEQ ID NO: 1), more preferably to Cys253 and Cys336, thereby inhibiting RBP activity of IGF2BP1.

More preferably, said anti-proliferative compound is a reversible or irreversible inhibitor of the proliferative activity of the IGF2 RBP. Reversible inhibitors comprise competitive inhibitors, non-competitive reversible inhibitors, slow-binding or tight-binding inhibitors, transition state analogues and multi-substrate analogues.

Competitive inhibitors show
i) non-covalent interactions with the RBP,
ii) compete with a substrate for the RBP binding site.

Non-competitive reversible inhibitors
i) bind at a site other than active site (allosteric binding site)
ii) cause a conformational change or introduce a modification in the RBP which decreases or stops the ability of the RBP to bind a substrate.
Irreversible inhibitors drive the equilibrium between the unbound RBP and inhibitor and the RBP-inhibitor-complex all the way to the side of the RBP-inhibitor-complex with a covalent bond (~100 kcal/mole), making the inhibition irreversible.

Preferred according to the present invention are irreversible inhibitors of the RBP.

Most preferred are covalent irreversible inhibitors of the RBP.

The term "RBP activity" as used herein is defined as the pathological, i.e. proliferative and/or pro-metastatic activity, the RBP exhibits in binding to a pathological binding partner that causes a pathological proliferation, tumor growth and/or metastasis.

### Potency of RBP inhibition

In light of the correlation with RBP inhibition, in preferred embodiments, the subject method and medical use utilize a compound (a) inhibiting the 2D proliferation of a tumor cell population with an IC₅₀ of 1 µM or less, even more preferably of 0.8 µM or less, or most preferably of 0.6 µM or less, and (b) significantly impairing (compared to vehicle controls) at least one of the three following properties: 1) 3D tumor cell spheroid growth; 2) matrigel invasion; 3) anoikis resistance. Indeed, inhibitors with IC₅₀ values in the lower micromolar, preferably the nanomolar and even more preferably the picomolar range are contemplated in (a), 2D proliferation. Thus, while the active agents are described herein, for convenience, as "RBP inhibitors", it will be understood that such nomenclature is not intended to limit the subject matter of the invention in any way.

### Molecular weight of RBP inhibitors

In general, the RBP inhibitors of the subject method or medical use will be small molecules, e.g., with molecular weights of 1000 g/mole or less, 500 g/mole or less, preferably of 400 g/mole or less, and even more preferably of 350 g/mole or less and even of 300 g/mole or less.

The term "subject" as used herein, refers to an animal, preferably a mammal, most preferably a human, who has been the object of treatment, observation or experiment and/or is suspected of being afflicted with a disease and/or condition as defined in the embodiments described herein.

The term "therapeutically effective amount" as used herein, means that amount of an active compound or a pharmaceutical agent that elicits the biological or medicinal response in a tissue system, animal or human being sought by a researcher, veterinarian, medical doctor or other clinician, which includes alleviation of the symptoms of the disease or disorder being treated.

As used herein, the term "pharmaceutically acceptable" embraces both human and veterinary use: for example the term "pharmaceutically acceptable" embraces a veterinary acceptable compound or a compound acceptable in human medicine and health care.

In a preferred embodiment, the anti-proliferative compound of the invention

### Pharmaceutically acceptable salts

In view of the close relationship between the free compounds and the compounds in the form of their salts or solvates, whenever a compound or inhibitor, respectively, is referred to in this context, a corresponding salt or solvate is also intended, provided such is possible or appropriate under the circumstances.

Salts and solvates of the inhibitors of the present invention and physiologically functional derivatives thereof which are suitable for use in medicine are those wherein the counter-ion or associated solvent is pharmaceutically acceptable. However, salts and solvates having non-pharmaceutically acceptable counter-ions or associated solvents are within the scope of the present invention, for example, for use as intermediates in the preparation of other compounds and their pharmaceutically acceptable salts and solvates.

Suitable salts according to the invention include those formed with both organic and inorganic acids or bases. Pharmaceutically acceptable acid addition salts include those formed from hydrochloric, hydrobromic, sulphuric, nitric, citric, tartaric, phosphoric, lactic, pyruvic, acetic, trifluoroacetic, triphenylacetic, sulphamic, sulphanilic, succinic, oxalic, fumaric, maleic, malic, mandelic, glutamic, aspartic, oxaloacetic, methanesulphonic, ethanesulphonic, arylsulphonic (for example p-toluenesulphonic, benzenesulphonic, naphthalenesulphonic or naphthalenedisulphonic), salicylic, glutaric, gluconic, tricarballylic, cinnamic, substituted cinnamic (for example, phenyl, methyl, methoxy or halo substituted cinnamic, including 4-methyl and 4-methoxycinnamic acid), ascorbic, oleic, naphthoic, hydroxynaphthoic (for example 1- or 3-hydroxy-2-naphthoic), naphthaleneacrylic (for example naphthalene-2-acrylic), benzoic, 4 methoxybenzoic, 2- or 4-hydroxybenzoic, 4-chlorobenzoic, 4-phenylbenzoic, benzeneacrylic (for example 1,4-benzenediacrylic), isethionic acids, perchloric, propionic, glycolic, hydroxyethanesulfonic, pamoic, cyclohexanesulfamic, salicylic, saccharinic and trifluoroacetic acid. Pharmaceutically acceptable base salts include ammonium salts, alkali metal salts such as those of sodium and potassium, alkaline earth metal salts such as those of calcium and magnesium and salts with organic bases such as dicyclohexylamine and N-methyl-D-glutamine.

All pharmaceutically acceptable acid addition salt forms of the inhibitors of the present invention are intended to be embraced by the scope of this invention.

Examples of solvates include hydrates.

### Polymorph crystal forms

Furthermore, some of the crystalline forms of the inhibitors may exist as polymorphs and as such are intended to be included in the present invention. In addition, some of the compounds may form solvates with water (i.e. hydrates) or common organic solvents, and such solvates are also intended to be encompassed within the scope of this invention. The inhibitors, including their salts, can also be obtained in the form of their hydrates, or include other solvents used for their crystallization.

### Prodrugs

The present disclosure further provides prodrugs of the inhibitors of this invention. In general, such prodrugs will be functional derivatives of the inhibitors, which are readily convertible in vivo into the desired therapeutically active inhibitors. Thus, in these cases, the methods of treatment disclosed herein, the term "administering" shall encompass the treatment of the various disorders described with prodrug versions of one or more of the itemed inhibitors, but which converts to the above specified inhibitors in vivo after administration to the subject. Conventional procedures for the selection and preparation of suitable prodrug derivatives are described, for example, in "Design of Prodrugs", ed. H. Bundgaard, Elsevier, 1985 and the patent applications DE 198 28 113, DE 198 28 114, WO 99/67228 and WO 99/67279.

### Protective Groups

During any of the processes for preparation of the inhibitors of the present invention, it may be necessary and/or desirable to protect sensitive or reactive groups on any of the molecules concerned. This may be achieved by means of conventional protecting groups, such as those described in Protective Groups in Organic Chemistry, ed. J.F.W. McOmie, Plenum Press, 1973; and T.W. Greene & P.G.M. Wuts, Protective Groups in Organic Synthesis, John Wiley & Sons, 1991. The protecting groups may be removed at a convenient subsequent stage using methods known from the art.

### Co-administration with other anticancer agents

The method comprises either co-administration of at least one RBP inhibitor and at least one of the other anti-cancer agents or the sequential administration thereof.

Co-administration includes administration of a formulation, which comprises at least one RBP inhibitor and at least one of the other anticancer agents or the essentially simultaneous administration of separate formulations of each agent.

Suitable other anticancer agents for co-administration with a RBP inhibitor may be selected from any FDA-or EMEA approved compounds suitable for cancer treatment. Some examples are doxorubicin and paclitaxel.

### Medical uses

Additionally, the present invention includes the use of the compounds of this invention and their corresponding pharmaceutically acceptable acid salt forms for the preparation of a medicament for the prevention or treatment of any of the above diseases or conditions.

Most preferably, the present RBP inhibitors are used for the prevention and/or treatment of malignant tumors of epithelial, mesenchymal, (neuro)-endocrine or hematopoietic origin. Even preferred is the use of the RBP inhibitors of the present invention for the prevention and/or treatment of cancer, wherein said cancer is selected from the group consisting of malignant tumors of epithelial, mesenchymal or haemopoietic cells, breast cancer, prostate cancer, pancreatic cancer, adrenal cancer, melanoma, lung cancer, colon cancer, leukemia (a liquid or non-solid tumor), soft tissue and bone sarcomas, neuroendocrine tumors such as islet cell carcinoma or medullary carcinoma of the thyroid, squamous carcinomas (particularly of the head and neck), adenocarcinomas, gliosarcomas such as glioblastoma multiforme, liver cancer, kidney cancer, bladder cancer, stomach cancer, thyroid carcinomas of follicular origin, neuroblastoma, teratoma and ovarian carcinoma. Most preferably the RBP inhibitors of the invention are used for the prevention and/or treatment of ovarian carcinoma, lung adenocarcinoma, melanoma, renal carcinoma, hepatocellular carcinoma, pancreas carcinoma, childhood-specific neuroblastoma and thyroid carcinoma. It has been shown that the RBP inhibitors of the present invention inhibit tumor metastasis. Accordingly, the RBP inhibitors of the invention are preferably used of the invention are used for the prevention and/or treatment of tumor metastasis or tumor recurrence.

### Pharmaceutical compositions and dose regimen

The compound may be administered to a patient by any conventional route of administration, including, but not limited to, intravenous, oral, subcutaneous, intramuscular, intradermal, parenteral and combinations thereof.

As used herein, the term "composition" is intended to encompass a product comprising the itemed compounds in the therapeutically effective amounts, as well as any product, which results, directly or indirectly, from combinations of the itemed compounds.

For liquid oral preparations, such as for example, suspensions, elixirs and solutions, suitable carriers and additives may advantageously include water, glycols, oils, alcohols, flavouring agents, preservatives, colouring agents and the like; for solid oral preparations such as, for example, powders, capsules, gelcaps and tablets, suitable carriers and additives include starches, sugars, diluents, granulating agents, lubricants, binders, disintegrating agents and the like.

Carriers, which can be added to the mixture, include necessary and inert pharmaceutical excipients, including, but not limited to, suitable binders, suspending agents, lubricants, flavorants, sweeteners, preservatives, coatings, disintegrating agents, dyes and colouring agents.

Soluble polymers as targetable drug carriers can include polyvinylpyrrolidone, pyran copolymer, polyhydroxypropylmeth-acrylamidephenol, polyhydroxyethylaspartamidephenol, or polyethyleneoxidepolyllysine substituted with palmitoyl residue(s). Furthermore, the inhibitors of the present invention may be coupled to a class of biodegradable polymers useful in achieving controlled/sustained release of a drug, for example, poly actic acid, poly-epsilon caprolactone, polyhydroxy butyric acid, polyorthoesters, polyacetals, polydihydropyrans, polycyanoacrylates and cross-linked or amphipathic block copolymers of hydrogels.

Suitable binders include, without limitation, starch, gelatin, natural sugars such as glucose or beta-lactose, corn sweeteners, natural and synthetic gums such as acacia, tragacanth or sodium oleate, sodium stearate, magnesium stearate, sodium benzoate, sodium acetate, sodium chloride and the like.

Disintegrating agents include, without limitation, starch, methyl cellulose, agar, bentonite, xanthan gum and the like.

Further, the present invention provides pharmaceutical compositions e.g. for parenteral, enteral or oral administration, comprising at least one RBP inhibitor, optionally in combination with at least one of other anticancer agent.

These combinations provide a particularly beneficial effect. Such combinations are therefore shown to be effective and useful for the treatment of cancer, tumor metastasis or tumor recurrence in a subject. Accordingly, the invention provides a method for the treatment of these conditions.

In a further preferred form of implementation, the invention relates to pharmaceutical compositions, that is to say, medicaments, that contain at least one compound of the invention or salts thereof, optionally in combination with one or more pharmaceutically acceptable carriers and/or solvents.

The pharmaceutical compositions may, for example, be in the form of parenteral or enteral formulations and contain appropriate carriers, or they may be in the form of oral formulations that may contain appropriate carriers suitable for oral administration. Preferably, they are in the form of oral formulations.

The inhibitors of RBP activity administered according to the invention may be employed in pharmaceutically administrable formulations or formulation complexes as inhibitors or in combination with inhibitors, substrates, pseudosubstrates, inhibitors of RBP expression, binding proteins or antibodies that reduce the RBP protein concentration in mammals. The compounds of the invention make it possible to adjust treatment individually to patients and diseases, it being possible, in particular, to avoid individual intolerances, allergies and side-effects.

The compounds also exhibit differing degrees of activity as a function of time. The physician providing treatment is thereby given the opportunity to respond differently to the individual situation of patients: he is able to adjust precisely, on the one hand, the speed of the onset of action and, on the other hand, the duration of action and especially the intensity of action.

The compounds may be advantageously administered, for example, in the form of pharmaceutical preparations that contain the active ingredient in combination with customary additives like diluents, excipients and/or carriers known from the prior art. For example, they can be administered parenterally (for example i.v. in physiological saline solution) or enterally (for example orally, formulated with customary carriers).

Depending on their endogenous stability and their bioavailability, one or more doses of the compounds can be given per day in order to achieve the desired reduction of RBP activity. For example, such a dosage range in humans may be in the range of from about 0.01 mg to 250.0 mg per day, preferably in the range of about 0.01 to 100 mg of compound per kilogram of body weight per day.

The compounds used according to the invention can accordingly be converted in a manner known per se into conventional formulations, such as, for example, tablets, (bitable) capsules, dragées, pills, suppositories, granules, aerosols, syrups, drops, liquid, solid and cream-like emulsions and suspensions and/or also as suppositories or as nasal sprays solutions, using inert, non-toxic, pharmaceutically suitable carriers and additives or solvents. In each of those formulations, the therapeutically effective compounds are preferably present in a concentration of approximately from 0.1 to 80% by weight, more preferably from 1 to 50% by weight, of the total mixture, that is to say, in amounts sufficient for the mentioned dosage latitude to be obtained.

The formulations may be advantageously prepared, for example, by extending the active ingredient with solvents and/or carriers, optionally with the use of emulsifiers and/or dispersants, it being possible, for example, in the case where water is used as diluent, for organic solvents to be optionally used as auxiliary solvents.

Examples of excipients useful in connection with the present invention include: water, non-toxic organic solvents, such as paraffins (for example natural oil fractions), vegetable oils (for example rapeseed oil, groundnut oil, sesame oil), alcohols (for example ethyl alcohol, glycerol), glycols (for example propylene glycol, polyethylene glycol); solid carriers, such as, for example, natural powdered minerals (for example highly dispersed silica, silicates), sugars (for example raw sugar, lactose and dextrose); emulsifiers, such as non-ionic and anionic emulsifiers (for example polyoxyethylene fatty acid esters, polyoxyethylene fatty alcohol ethers, alkylsulphonates and arylsulphonates), dispersants (for example lignin, sulphite liquors, methylcellulose, starch and polyvinylpyrrolidone) and lubricants (for example magnesium stearate, talcum, stearic acid and sodium lauryl sulphate) and optionally flavourings.

Administration may be carried out in the usual manner, preferably enterally or parenterally, especially orally. In the case of enteral administration, tablets may contain in addition to the mentioned carriers further additives such as sodium citrate, calcium carbonate and calcium phosphate, together with various additives, such as starch, preferably potato starch, gelatin and the like. Furthermore, lubricants, such as magnesium stearate, sodium lauryl sulphate and talcum, can be used concomitantly for tableting. In the case of aqueous suspensions and/or elixirs intended for oral administration, various taste correctives or colorings can be added to the active ingredients in addition to the above-mentioned excipients.

In the case of parenteral administration, solutions of the active ingredients using suitable liquid carriers can be employed. In general, it has been found advantageous to administer, in the case of intravenous administration, amounts of approximately from 0.01 to 2.0 mg/kg, preferably approximately from 0.01 to 1.0 mg/kg, of body weight per day to obtain effective results and, in the case of enteral administration, the dosage is approximately from 0.01 to 2 mg/kg, preferably approximately from 0.01 to 1 mg/kg, of body weight per day.

It may nevertheless be necessary in some cases to deviate from the stated amounts, depending upon the body weight of the experimental animal or the patient or upon the type of administration route, but also on the basis of the species of animal and its individual response to the medicament or the interval at which administration is carried out. Accordingly, it may be sufficient in some cases to use less than the above-mentioned minimum amount, while, in other cases, the mentioned upper limit will have to be exceeded. In cases where relatively large amounts are being administered, it may be advisable to divide those amounts into several single doses over the day. For administration in human medicine, the same dosage latitude is provided. The above remarks apply analogously in that case.

In another aspect, the invention provides a method for identifying non-catalytic cysteine residues in RNA-binding proteins. Said method preferably comprises the following steps:
a. Selecting a RNA-binding protein,
b. Recombinant expression and/or purification of the selected RNA-binding protein,
c. Posttranslational chemical mutagenesis of the RNA-binding protein *in vitro* using cysteine-directed probes,
d. Limited proteolysis of the chemically modified RNA-binding protein and analysis of the resulting peptide solution for modified cysteine residues,
e. *In vitro* functional assays to assess the activity of the modified cysteine residues of the RNA-binding protein;
f. Site-directed mutagenesis to produce a mutant version of the RNA-binding protein and assessment of the function *in vitro.*

The RNA-binding protein used in the method of the invention is preferably a protein of the IGF2 family, more preferably selected from IGF2BP1, IGF2BP2 and IGF2BP3. Most preferably, the RNA-binding protein used in the method of the invention is IGF2BP1.

The recombinant expression and/or purification of the selected RNA-binding protein according to step b. can be performed using standard methodologies, which are well known to the person skilled in the art.

As for step c., posttranslational chemical mutagenesis of the RNA-binding protein *in vitro* using cysteine-directed probes can be performed as described herein in *Example 1* below.

Limited proteolysis of the chemically modified RNA-binding protein and analysis of the resulting peptide solution for modified cysteine residues according to step d. can be performed using any suitable method, e.g. as described herein in *Examples 1 and*/*or 2* below. A suitable method for the analysis of the resulting peptide solution is for example LC-MS/MS spectrometry.

*In vitro* functional assays of step e. to assess the activity of the modified cysteine residues of the RNA-binding protein can be performed using any methods known to the person skilled in the art. A suitable method is e.g. described herein in Example 3 below.

Methods to perform site-directed mutagenesis of step f. to produce a mutant version of the RNA-binding protein and assessment of the function *in vitro* and/or *in cellulo* are generally known to the person skilled in the art. A suitable method to perform site-directed mutagenesis is e.g. described herein in *Example* 4 below. The *in vitro* and/or *in cellulo* assessment of the function of a target in cancer development is usually performed in cancer cell models. Suitable cell lines as cancer cell models include ES-2, A549, MV3, 786-O, Huh7, PANC1, H1975, BE2C, Kelly, C643, and 8305C. Preferred according to the invention are ES-2, A549, MV3, 786-O, Huh7 and PANC1. Most preferred is an ovarian carcinoma-derived ES-2 cell line. A suitable method to perform the *in vitro* and/or *in cellulo* assessment of the function of a RNA-binding protein of the invention in cancer development is e.g. described herein in *Example 5* below.

The application further discloses an *in vivo* assessment of the role of the identified cysteine residue(s) in the intracellular activity of the RNA-binding protein, whichis usually performed in xenograft mouse models, which are generally known to the person skilled in the art. A suitable method to perform the *in vivo* assessmentis e.g. described in *Example* 6 below.

In a further aspect, the invention provides an *in vitro* screening method for identifying an anti-proliferative compound for use in the prevention or treatment of cancer, tumor metastasis or tumor recurrence in a subject, said *in vitro* method comprising the steps of:
a. Selecting a RNA-binding protein, wherein said RNA-binding protein is a IGF2 mRNA-binding protein,
b. Providing cells of a cancer cell model, wherein the cells of said cancer cell model express the IGF2 mRNA-binding protein;
c. Providing the same cancer cell model as in step b., wherein the gene of the IGF2 mRNA-binding protein has been knocked-out in the cells of said cancer cell model or wherein the cells of said cancer cell model express a mutant, non-functional IGF2 mRNA-binding protein;
d. Contacting the cells of steps b. and c. with a test compound,
e. Measuring at least one of the 3D tumor cell spheroid growth, matrigel invasion and anoikis resistance (self-renewal) of the cells contacted with the test compound according step d.,
f. Selecting a test compound as an anti-proliferative compound for use in the prevention or treatment of cancer, tumor metastasis or tumor recurrence, when said test compound leads in the cells of step b., when compared to the cells of step c., to the inhibition of at least one of 3D tumor cell spheroid growth, matrigel invasion and anoikis resistance.

The RNA-binding protein used in this screening method of the invention is preferably a protein selected from IGF2BP1, IGF2BP2 and IGF2BP3. Most preferably, the RNA-binding protein used in the method of the invention is IGF2BP1.

In a most preferred embodiment of the screening method of the invention, the cells of step c. express a mutant, non-functional IGF2BP1, which comprises mutations of the non-catalytic cysteine residues C253A and C336A.

Suitable cell lines as cancer cell models for steps b. and c. of the screening method include ES-2, A549, MV3, 786-O, Huh7, PANC1, H1975, BE2C, Kelly, C643, and 8305C. Preferred according to the invention are ES-2, A549, MV3, 786-O, Huh7 and PANC1. Most preferred is an ovarian carcinoma-derived ES-2 cell line.

The cells of step b. of the screening method preferably express either endogenously or recombinantly an IGF2 mRNA-binding protein, most preferably IGF2BP1.

The screening method for identifying an anti-proliferative compound can be performed e.g. as described in *Example 7, 8 and 9* below.

Described herein is an advantageous approach to discover anti-proliferative compounds which are capable to inhibit RNA binding proteins of the IGF2 family. Small chemical probes that target chemo- and regio-selectively cysteines can reveal a hitherto unknown information about the function of the residues they preferentially modify (Bertoldo et al., 2017; Bertoldo et al., 2018). Since these residues play a critical role in the structure-related function of the selected protein, they were used as targets in a drug discovery program. This information can help to direct the efforts towards a functionally relevant site-region or residue in the protein target, thus allowing the development of innovative covalent inhibitors for non-catalytic proteins. In the case of IGF2BP1 it was surprisingly found that the preferentially modified residues Cys253 and Cys336 are essential for the cancer progression promoted by IGF2BP1 in ovarian carcinoma and other cancer entities. They are likely implicated in KH1-2 domains' ability to create a structural platform that leads to specific RNA recognition and remodeling (Dagil et al., 2019), thus facilitating IGF2BP1 binding and upregulation of oncogenic mRNAs(Busch et al., 2016; Hamilton et al., 2013; Muller et al., 2018; Muller et al., 2019; Tessier et al., 2004). By being involved in the progression of several different cancer entities, including ovarian carcinoma (Kobel et al., 2007; Muller et al., 2018), IGF2BP1 serves as promising target in cancer therapeutics, especially due to its oncofetal expression patterns (Bell et al., 2013). Despite its essential role, there has been no progress in the design of specific inhibitors in the prior art so far. In fact, only a handful of molecules have been discovered so far as able to target RBPs (Mahapatra et al., 2017; Minuesa et al., 2019; Roos et al., 2016; Wu et al., 2015), mostly via high-throughput screening assays (HTS). The lack of a specific drug design platform in RBPs is attributed to the usual intrinsic problems in targeting non-catalytic proteins. Accordingly, the platform of the invention overcomes the limitations of HTS assays by providing a fast and reliable avenue to specifically target RBPs. Firstly, it is revealed which amino acid (in the present case cysteine) is functionally relevant, thus worth to target. Next, a search for small chemical probes that can covalently modify those residues intracellularly, is performed, whilst functioning as inhibitors of RBP-induced cancer phenotypes. In addition, the identified residues can also be used as templates in virtual screening assays, reducing considerably the amount of time and work necessary to find a good drug lead. This rationale was used with IGF2BP1's Cys253 and Cys336 and thereby, the compound *(E)-N*-ethyl-4-oxo-4-phenylbut-2-enamide (designated as J5 in the examples of the invention), a cysteine-directed covalent inhibitor that displays a potent anti-tumor activity with no systemic toxicity, was discovered. *(E)-N*-ethyl-4-oxo-4-phenylbut-2-enamide impairs ovarian carcinoma progression by targeting IGF2BP1 in a Cys253/Cy336-specific manner, leading to a drastic reduction in tumor burden. *(E)-N*-ethyl-4-oxo-4-phenylbut-2-enamide is also potent in other types of cancer where this protein is *de novo* synthesized and emerges as promising new drug in a targeted cancer therapy.

The above disclosure describes the present invention in general. A more complete understanding can be obtained by reference to the following figures and examples.

### Examples of the Invention

### Example 1: Site-specific protein modification

### Methodology

A multiple-Cys-containing RNA-binding protein with a recognized role in cancer is selected, for example and not limited to, the Insulin-like growth factor 2 mRNA binding protein 1 (IGF2BP1). It is then expressed in bacteria (not limited to), purified and submitted to site-specific protein modification reactions in vitro using cysteine-directed probes (Cys-to-Dha conversion), for example and not limited to, the compound 2,5 dibromohexanediamide **(1).** The reactions are performed in mild conditions, PBS buffer pH 8.0 for 1 h at 37∘C, using increasing equivalents of **1.** The resulting reaction is then purified with a spin column and submitted to trypsin digestion followed by LC-MS/MS using standard protocols. The identification of Dha-modified peptides is performed by adding the corresponding mass shift of 33.9878 m/z (Dha) as a post-translational modification in the global search for modifications. The Dha conversion of specific cysteine residues is located within the primary sequence of the protein of interest (POI) to uncover preferentially modified cysteine residues.

### Results

Accordingly, two cysteines residues were found to be preferentially modified by the compound 1 following a ramp of increasing concentrations. Cys253 was modified with 200 eq of 1 and Cys336 with 300 eq of 1. It is important to note that in some reactions Cys336 was also modified with 200 eq of 1, which indicates a similar reactivity with Cys253. Thus, these two cysteines were the preferentially modified residues by the site-specific protein modification protocol (Figure 2). By being preferentially modified, these residues are considered to be the most reactive cysteine residues in IGF2BP1, which according to previous data (Bertoldo et al., 2017; Bertoldo et al., 2018) are likely to be functionally relevant.

### Example 2: Structural analysis of preferentially modified cysteine residues in IGF2BP1

### Methodology

Crystal structure of IGF2BP1 KH domains 1 and 2 (PDB 6QEY) was used in 1 uS molecular dynamics simulations to analyze pKa values to corroborate the chemical modifications.

### Results

Figure 3 shows that the preferentially modified residues Cys253 and Cys336 have relatively similar pKas in the range of 10.5-10.9. At concentrations of 200 or 300 eq, only modification of Cys253 and Cys336 was observed. At higher concentrations of 2,5 dibromohexanediamide **(1)** (above 500 eq) almost all cysteines the exception of Cys110 (see Figure 2) were modified *in vitro.* These findings suggest that Cys253 and Cys336 show the highest reactivity for **1.**

### Example 3: Functional analysis of preferentially modified residues

### Methodology

In order to show that the preferentially modified residues Cys253 and Cys336 are functionally relevant a series of site-directed mutagenesis reactions were performed. KH 1-4 truncated versions of IGF2BP1 wild type, site-directed mutant C253/336A (genetic mutant) and chemical mutant Dha253/336 were used for RNA-binding and structural assays. RNA binding assays were performed with RNA oligo substrates derived from known IGF2BP1 mRNA targets (LIN28B and Myc). These oligos were designed and purchased with or without a 5'FAM label to be used in fluorescent polarization/(de)quenching assays and circular dichroism (CD) analyses.

### Results

Both mutants present significant lower K_{D} values, 105 nM for the Dha253/336A mutant and 35 nM for the C253/336A mutant (Figure 4A), compared to the wild type protein (K_{D} ~11nM). Moreover, a rather higher proportion of RNA/protein (1:5 Dha253/336, 1:9 C253/336A) is required to reach maximum intensity in the (de)quenching assay, compared to the wild type protein (1:1 ratio of protein/RNA). Taken together, these data demonstrate that Cys253 and Cys336 are functionally relevant for the RNA-binding activity of IGF2BP1 and that their perturbation impairs the affinity of protein-RNA association. Structural analyses by CD revealed that the mutation of Cys253 and Cys336 to alanine did not cause and overall change in the conformation of IGF2BP1's KH1-4 domain suggesting only modest and locally restricted structural rearrangements (Figure 4B, C). These findings strengthen the argument that Cys253 and Cys336 are directly involved in IGF2BP1-RNA association, contributing to the affinity and/or stability of complex formation.

### Example 4: Functional relevance of Cys253 and 336 for IGF2BP1's intracellular activity

### Methodology

To assess the role of the identified cysteine residues in the intracellular activity of the target RBP, a cDNA (coding DNA) insert containing the Cys-to-Ala mutation was produced and cloned into a eukaryotic expression vector. The vectors were also designed to contain an SBP-Flag tag (i.e in pCDNA 3.1) or a GFP tag, (i.e pLVX) to trace the protein in cells or isolate it by affinity purification. The vector containing the cysteine mutated target RBP was then transfected or transduced (stable insertion) into the cancer cell lines of interest, here the ovarian carcinoma-derived ES-2 cell line for the prove of concept. In the recipient cell line the wild type protein had been deleted by CRISPR/Cas9 technologies (Muller et al., 2018). This cellular setting allows analyzing the phenotypic roles of the mutant protein, here comprising Cys-Ala conversion of previously identified residues (Cys 253 and Cys336 in IGF2BP1), in comparison to the wild type protein or an unrelated protein like GFP, serving as negative control. The successful expression of these proteins of interest (POIs) in the cell line of interest (COI, here ES-2 cells) was validated at the mRNA and protein levels using RT-qPCR and Western blotting, respectively (data not shown).

### Results

Data derived from RT-qPCR and Western blot analyses revealed that residues Cys253 and Cys336 are also important for the intracellular RNA-binding activity of IGF2BP1. This is indicated by impaired RNA-dependent protein-protein association of IGF2BP1 with ELAVL1 and hnRNPU (Figure 5A, B; (Busch et al., 2016; Wachter et al., 2013; Weidensdorfer et al., 2009)) as well as reduced binding to the non-coding Y3 RNA (Figure 5C; (Kohn et al., 2010)). Notably, these findings suggest that the identified cysteines residues do not only modulate specific RNA-associations of IGF2BP1 but rather serve a conserved role in IGF2BP1-RNA association as well as the RNA-dependent association of IGF2BP1 with regulatory mRNPs.

In previous studies, it was demonstrated that the depletion or deletion of IGF2BP1 promotes the miRNA-directed decay of various IGF2BP1-associated mRNAs, including HMGA2 and LIN28B (Busch et al., 2016). In contrast to IGF2BP1-KO cells stably expressing GFP (GFP-only), RNA-binding deficient IGF2BP1 (GFP-KHmut) or GFP-IGF2BP1_{C253/336A} (GFP-C253/336A), the re-expression of GFP-tagged wild type IGF2BP1 (GFP-I1) substantially enhanced the protein abundance of HMGA2 and LIN28B (Figure 5D, E). Notably, the Cys253/336 mutant protein still enhanced protein abundance although significantly less pronounced compared to wild type IGF2BP1. In conclusion these findings indicate that mutation of Cys253/336 impairs the main role of IGF2BP1 in cancer-derived cells, the post-transcriptional enhancement of oncogenic factors, here HMGA2 and LIN28B.

### Example 5: The role of Cys253/336 in IGF2BP1-enhanced tumor cell properties

### Methodology

To assess the role of Cys253/336 in IGF2BP1-enhanced cancer cell properties, 3D tumor cell spheroid growth, matrigel invasion and anoikis resistance (self-renewal) were analyzed in ES-2 IGF2BP1-KO cells stably re-expressing GFP (GFP-only; serving as control) wild type GFP-tagged IGF2BP1 (GFP-I1) or the GFP-IGF2BP1_{C253/336A} mutant (GFP-C253/336A). Notably, in previous studies, it was demonstrated that IGF2BP1 promotes all three parameters in ES-2 as well as other cancer-derive cells (Busch et al., 2016; Muller et al., 2018; Muller et al., 2019).

### Results

The non-adhesive 3D spheroid growth of IGF2BP1-KO ES-2 cells was substantially enhanced by the expression of GFP-IGF2BP1 whereas growth was significantly lower in cells expressing the genetic GFP-C253/336A mutant (Figure 6A, B; spheroid viability). The matrigel invasion of ES-2 cells was markedly elevated by the expression of wild type IGF2BP1 and only modestly promoted by the genetic mutant (Figure 6A, B; spheroid invasion and invasion index). The most severe difference was observed for anoikis resistance. Only the expression of wild type IGF2BP1 enhanced anoikis resistance (Figure 6A, B; anoikis resistance). GFP controls and cells expressing the genetic mutant were indistinguishable and showed significantly reduced anoikis resistance compared to cells expressing the wild type IGF2BP1.

### Example 6: Cys253/336 are essentially involved in IGF2BP1-induced tumor growth

### Methodology

To evaluate the effect of the identified cysteine residue in IGF2BP1-induced tumor growth, IGF2BP1-KO ES-2 cells re-expressing the GFP-tagged wild type (GFP-I1) or the GFP-tagged Cys253/336Ala mutant were stably transduced with a tracer molecule, here iRFP (near-infrared fluorescent protein). The latter allows the tracing of tumor cells by non-invasive *in vivo* imaging (Gutschner et al., 2014; Muller et al., 2018). Cells (~1x10⁵ cells per mouse) were then injected subcutaneously (sc) in nude mice to monitor the impact of wild type versus Cys253/336Ala mutant IGF2BP1 in tumor growth.

### Results

In comparison to ES-2 cells re-expressing the wild type protein (GFP-I1), cells re-expressing the Cys253/336 mutant protein showed reduced tumor growth over time resulting in significantly lower final tumor volume and mass (Figure 7A-E). These findings clearly indicate that Cys253/336 are essential for IGF2BP1-driven tumor growth in experimental tumor models. Notably, the observed difference between the wild type and mutant protein appeared substantially pronounced *in vivo* when compared to spheroid growth in vitro (see Figure 6). This showed that the role of Cys253/336 is of pivotal importance for the oncogenic tumor-promoting role of IGF2BP1 *in vivo.*

### Example 7: The Cys253/336 targeting compound J5 impairs tumor cell vitality

### Methodology

The drastically reduced tumor size observed for ES-2 cells expressing the Cys253/336 mutant IGF2BP1 revealed a hitherto unknown oncogenic activity of non-catalytic cysteines in an oncofetal RBP. This reveals how the method of the invention can be applied successfully to unraveling candidate target cysteines for therapeutic intervention in cancer treatment. It was now tested, whether targeting the identified residues with cysteine-directed small molecules can produce a promising anti-tumor therapy, as the effects of such cysteine-specific RBP inhibitor can resemble the same deleterious effects as the C253/336A mutation produces on IGF2BP1-induced tumor growth.

To probe if the identified residues are druggable targets, a search for chemical probes with exquisite chemo- and regioselectivity to cysteine residues was performed and their ability to target IGF2BP1's Cys253 and Cys336 was tested, whilst functioning as inhibitors. Two chemical probes were selected; a *(E)-N*-ethyl-4-oxo-4-phenylbut-2-enamide, **(herein designated as J5)** and its fluorescent derivative the compound *(E)-N-(2-((7-*nitrobenzo[c][1,2,5]oxadiazol-4-yl)amino)ethyl)-4-oxo-4-phenylbut-2-enamide(Bernardim et al., 2016) **(herein designated as J2).** Both probes are known to covalently modify cysteine residues in a highly site-specific manner. Previously, these probes were used to reveal a functionally relevant cysteine in a chaperone (Lindstedt et al., 2019) and to modulate its activity to produce an enhanced, not impaired, Hsp70 variant (known as Hsp70-caaNBF), able to act as an anti-aggregating enzyme *in vivo.* These probes were then used in ES-2 IGF2BP1 KO cells, re-expressing either the wild type version of IGF2BP1 or the C253/336A mutant. The initial assay was set to observe if the probes influence the 2D growth, determined here as cell density, of ES-2 cells in a IGF2BP1-Cys253/336-dependent manner. IC₅₀ values were determined by measuring the cell viability or cell confluence of cells incubated with increasing concentrations of J2 and J5.

### Results

At a concentration of 1µM, J2 demonstrated a strong impairment in the growth of ES-2 cells re-expressing the wild type protein (Figure 8A). This reduction was similar to the effect observed by re-expressing the C253/336A mutant in the absence of the drug. At a concentration of 1µM, J2 had no substantial impact on the growth of cells expressing the C253/336A mutant (Figure 8B). In agreement, the determination of IC₅₀ values for J2 revealed an IC50 of 0.84 µM for cells expressing wild type IGF2BP1, whereas the IC₅₀ value increased to 2.2 µM in ES-2 cells expressing the C253/336A mutant (Figure 8C). These features provide strong evidence that Cys253/336 might be intracellular targets of J2 and that their covalent modification by J2 impairs IGF2BP1-driven tumor cell proliferation.

### Example 8: The "drug-likeness" of chemical probes J2 and J5

### Methodology

J2's rather unexpected 2-fold selective cytoreductive effect prompted us to further investigate its activity in order to confirm IGF2BP1 target engagement. Unfortunately, due to solubility problems J2 failed in the "drug-likeness" assessment. It was then decided to evaluate J5 activity since it has the same scaffold and cysteine-reactivity, but without the fluorescent moiety. Cell vitality was assessed by Cell Titer Glo (Promega) in these analyses.

### Results

Interestingly, J5 also presented a strong cytoreductive effect as J2 with as much as 1.8-fold selectivity to ES-2 cells re-expressing the wild type IGF2BP1 (IC50_{WT} 0.97 µM, IC50_{C253/336A} 1.75 µM). Thus, indicating that as J2, J5 might be targeting Cys253 and Cys336 in a preferential manner (Figure 9A,B), however with a much higher solubility in the assays. J5 selective effect is also observed in the invasion assays performed with ES-2 IGF2BP1 KO cells. Figure 9E and F show that once more, cells re-expressing IGF2BP1 without the cysteine target residues (GFP-IGF2BP1_{C253/336A}) were strongly resistant to J5 treatment and displayed an unchallenged invasion up to 6 µM. It is important to note that a variant of J5 without its Michael acceptor portion (J5 (-)) is completely inactive and has no effect on the viability of the tested cells (Figure 9C). This data reinforces the IGF2BP1 cysteine-dependency of J5 inhibitory activity and corroborates Cys253/Cys336 as its irreversible intracellular targets.

Consequently, J5 potency was further tested in naive ES-2 cells, which express the native form of IGF2BP1 and compared to ES-2 cells with IGF2BP1 deletion. Once again J5 showed around 1.8-fold selectivity to naive ES-2 cells when compared to the IGF2BP1 deleted ES-2 cells (Figure 9A) and a strong cytoreductive effect (0.58 µM). J5 potency was further evaluated in a panel of cancer cell lines and demonstrated potency as anti-cancer drug in all of them (Figure 9D). Taken together, these results highlight J5 as promising cysteine-directed IGF2BP1-targeting small molecule with a strong anti-cancer activity and selectivity. Its rather small size and chemical features will enable its optimization and application in a targeted cancer therapy.

### Example 9: J5 is a potent IGF2BP1-targeting anti-tumor agent

### Methodology

Ovarian carcinoma-derived ES-2 cells re-expressing the wild-type IGF2BP1 (GFP-IG2BP1) or the C253/336A mutant (GFP-IG2BP1_{C253/336A}) were injected in the left flank of female SCID mice (1-5 x 10⁵ cells). Tumor growth was monitored for 7 days and treatments started when tumors reached ~ 35 mm³. Mice were randomized into 5 groups of 6 animals. Groups were treated daily with saline or saline + J5 [0.1 mg/kg] for GFP-IG2BP1 and GFP-IGF2BPI_{C253/336A}-expressing tumors. Saline or saline + J5(-) [0.1 mg/kg] for GFP-IG2BP1-expressing tumors intraperitoneally.

### Results

J5 demonstrated a strong anti-tumor effect against xenografts bearing ES-2 cells re-expressing the wild-type IGF2BP1 (GFP-IGF2BP1) with no systemic toxicity (Figure 10A-C and J). Nevertheless, on tumors bearing ES-2 cells re-expressing the C253/336A mutant (GFP-IG2BP1_{253/336A}) it had absolutely no effect, providing a compelling evidence of its *in vivo* selectivity to IGF2BP1's functionally relevant residues Cys253 and Cys336 (Figure 10 D-F). Furthermore, the anti-tumor effect of J5 is linked to the covalent modification of Cys253 and Cys336, since its non-Michael acceptor version had also no impact on the progression of wild type GFP-IGF2BP1 re-expressing tumors (Figure 10G-I). Taken together, these results provide a clear picture of how the cysteine-directed covalent modulation of IGF2BP1 impairs ovarian carcinoma progression.

### References

Bell, J. L., Turlapati, R., Liu, T., Schulte, J. H., and Huttelmaier, S. (2015). IGF2BP1 harbors prognostic significance by gene gain and diverse expression in neuroblastoma. J Clin Oncol 33, 1285-1293.
Bell, J. L., Wachter, K., Muhleck, B., Pazaitis, N., Kohn, M., Lederer, M., and Huttelmaier, S. (2013). Insulin-like growth factor 2 mRNA-binding proteins (IGF2BPs): post-transcriptional drivers of cancer progression? Cell Mol Life Sci 70, 2657-2675.
Bernardim, B., Cal, P. M., Matos, M. J., Oliveira, B. L., Martinez-Saez, N., Albuquerque, I. S., Perkins, E., Corzana, F., Burtoloso, A. C., Jimenez-Oses, G., and Bernardes, G. J. (2016). Stoichiometric and irreversible cysteine-selective protein modification using carbonylacrylic reagents. Nat Commun 7, 13128.
Bertoldo, J. B., Rodrigues, T., Dunsmore, L., Aprile, F. A., Marques, M. C., Rosado, L. A., Boutureira, O., Steinbrecher, T. B., Sherman, W., Corzana, F., et al. (2017). A Water-Bridged Cysteine-Cysteine Redox Regulation Mechanism in Bacterial Protein Tyrosine Phosphatases. Chem 3, 665-677.
Bertoldo, J. B., Terenzi, H., Huttelmaier, S., and Bernardes, G. J. L. (2018). Posttranslational Chemical Mutagenesis: To Reveal the Role of Noncatalytic Cysteine Residues in Pathogenic Bacterial Phosphatases. Biochemistry 57, 6144-6152.
Busch, B., Bley, N., Muller, S., Glass, M., Misiak, D., Lederer, M., Vetter, M., Strauss, H. G., Thomssen, C., and Huttelmaier, S. (2016). The oncogenic triangle of HMGA2, LIN28B and IGF2BP1 antagonizes tumor-suppressive actions of the let-7 family. Nucleic Acids Res 44, 3845-3864.
Dagil, R., Ball, N. J., Ogrodowicz, R. W., Hobor, F., Purkiss, A. G., Kelly, G., Martin, S. R., Taylor, I. A., and Ramos, A. (2019). IMP1 KH1 and KH2 domains create a structural platform with unique RNA recognition and re-modelling properties. Nucleic Acids Res.
Degrauwe, N., Suva, M. L., Janiszewska, M., Riggi, N., and Stamenkovic, I. (2016). IMPs: an RNA-binding protein family that provides a link between stem cell maintenance in normal development and cancer. Genes Dev 30, 2459-2474.
Gutschner, T., Hammerle, M., Pazaitis, N., Bley, N., Fiskin, E., Uckelmann, H., Heim, A., Grobeta, M., Hofmann, N., Geffers, R., et al. (2014). Insulin-like growth factor 2 mRNA-binding protein 1 (IGF2BP1) is an important protumorigenic factor in hepatocellular carcinoma. Hepatology 59, 1900-1911.
Hamilton, K. E., Noubissi, F. K., Katti, P. S., Hahn, C. M., Davey, S. R., Lundsmith, E. T., Klein-Szanto, A. J., Rhim, A. D., Spiegelman, V. S., and Rustgi, A. K. (2013). IMP1 promotes tumor growth, dissemination and a tumor-initiating cell phenotype in colorectal cancer cell xenografts. Carcinogenesis 34, 2647-2654.
Kobel, M., Weidensdorfer, D., Reinke, C., Lederer, M., Schmitt, W. D., Zeng, K., Thomssen, C., Hauptmann, S., and Huttelmaier, S. (2007). Expression of the RNA-binding protein IMP1 correlates with poor prognosis in ovarian carcinoma. Oncogene 26, 7584-7589.
Kohn, M., Lederer, M., Wachter, K., and Huttelmaier, S. (2010). Near-infrared (NIR) dye-labeled RNAs identify binding of ZBP1 to the noncoding Y3-RNA. RNA 16, 1420-1428.
Lindstedt, P. R., Aprile, F. A., Matos, M. J., Perni, M., Bertoldo, J. B., Bernardim, B., Peter, Q., Jimenez-Oses, G., Knowles, T. P. J., Dobson, C. M., et al. (2019). Enhancement of the Anti-Aggregation Activity of a Molecular Chaperone Using a Rationally Designed Post-Translational Modification. Acs Central Sci 5, 1417-1424.
Mahapatra, L., Andruska, N., Mao, C., Le, J., and Shapiro, D. J. (2017). A Novel IMP1 Inhibitor, BTYNB, Targets c-Myc and Inhibits Melanoma and Ovarian Cancer Cell Proliferation. Transl Oncol 10, 818-827.
Minuesa, G., Albanese, S. K., Xie, W., Kazansky, Y., Worroll, D., Chow, A., Schurer, A., Park, S. M., Rotsides, C. Z., Taggart, J., et al. (2019). Small-molecule targeting of MUSASHI RNA-binding activity in acute myeloid leukemia. Nat Commun 10, 2691.
Muller, S., Bley, N., Glass, M., Busch, B., Rousseau, V., Misiak, D., Fuchs, T., Lederer, M., and Huttelmaier, S. (2018). IGF2BP1 enhances an aggressive tumor cell phenotype by impairing miRNA-directed downregulation of oncogenic factors. Nucleic Acids Res 46, 6285-6303.
Muller, S., Glass, M., Singh, A. K., Haase, J., Bley, N., Fuchs, T., Lederer, M., Dahl, A., Huang, H., Chen, J., et al. (2019). IGF2BP1 promotes SRF-dependent transcription in cancer in a m6A- and miRNA-dependent manner. Nucleic Acids Res 47, 375-390.
Roos, M., Pradere, U., Ngondo, R. P., Behera, A., Allegrini, S., Civenni, G., Zagalak, J. A., Marchand, J. R., Menzi, M., Towbin, H., et al. (2016). A Small-Molecule Inhibitor of Lin28. ACS Chem Biol 11, 2773-2781.
Tessier, C. R., Doyle, G. A., Clark, B. A., Pitot, H. C., and Ross, J. (2004). Mammary tumor induction in transgenic mice expressing an RNA-binding protein. Cancer Res 64, 209-214.
Wachter, K., Kohn, M., Stohr, N., and Huttelmaier, S. (2013). Subcellular localization and RNP formation of IGF2BPs (IGF2 mRNA-binding proteins) is modulated by distinct RNA-binding domains. Biol Chem 394, 1077-1090.
Weidensdorfer, D., Stohr, N., Baude, A., Lederer, M., Kohn, M., Schierhorn, A., Buchmeier, S., Wahle, E., and Huttelmaier, S. (2009). Control of c-myc mRNA stability by IGF2BP1-associated cytoplasmic RNPs. RNA 15, 104-115.
Wu, X., Lan, L., Wilson, D. M., Marquez, R. T., Tsao, W. C., Gao, P., Roy, A., Turner, B. A., McDonald, P., Tunge, J. A., et al. (2015). Identification and validation of novel small molecule disruptors of HuR-mRNA interaction. ACS Chem Biol 10, 1476-1484.

## Claims

1. An anti-proliferative compound or a pharmaceutical composition comprising said anti-proliferative compound for use in the prevention or treatment of cancer, tumor metastasis or tumor recurrence in a subject, wherein said anti-proliferative compound binds covalently to at least one non-catalytic cysteine residue of an IGF2 (insulin-like growth factor 2) mRNA-binding protein and thereby inhibits the proliferative activity of said IGF2 mRNA-binding protein, **characterized in that** said anti-prolifrative compound is selected from *(E)-N-*(2-((7-nitrobenzo[c][1,2,5]oxadiazol-4-yl)amino)ethyl)-4-oxo-4-phenylbut-2-enamide and (E)-N-ethyl-4-oxo-4-phenylbut-2-enamide, including pharmaceutically acceptable salt, solvate or polymorph thereof, including all tautomers and stereoisomers thereof

2. The anti-proliferative compound or pharmaceutical composition for use according to claim 1, wherein said IGF2 mRNA-binding protein (IGF2BP) is selected from IGF2BP1, IGF2BP2 and IGF2BP3

3. The anti-proliferative compound or pharmaceutical composition for use according to claim 1 or 2, wherein said RNA-binding protein is IGF2BP1.

4. The anti-proliferative compound or pharmaceutical composition for use according to any one of the preceding claims, wherein said anti-proliferative compound selectively binds to the non-catalytic cysteine residue(s) Cys253 and/or Cys336 of IGF2BP1, when referring to the polypeptide which has the amino acid sequence of SEQ ID NO: 1.

5. The anti-proliferative compound or pharmaceutical composition for use according to any one of the preceding claims, wherein said proliferative disease is selected from malignant tumors of epithelial, mesenchymal, (neuro)-endocrine or hematopoietic origin

6. The anti-proliferative compound or pharmaceutical composition for use according to any one of the preceding claims, wherein said proliferative disease is cancer and is selected from the group consisting of malignant tumors of epithelial, mesenchymal or haemopoietic cells, breast cancer, prostate cancer, pancreatic cancer, adrenal cancer, melanoma, lung cancer, colon cancer, leukemia (a liquid or non-solid tumor), soft tissue and bone sarcomas, neuroendocrine tumors such as islet cell carcinoma or medullary carcinoma of the thyroid, squamous carcinomas (particularly of the head and neck), adenocarcinomas, gliosarcomas such as glioblastoma multiforme, liver cancer, kidney cancer, bladder cancer, stomach cancer, thyroid carcinomas of follicular origin, neuroblastoma, teratoma and ovarian carcinoma; preferably ovarian carcinoma, lung adenocarcinoma, melanoma, renal carcinoma, hepatocellular carcinoma, pancreas carcinoma and childhood-specific neuroblastoma.

7. The anti-proliferative compound or pharmaceutical composition for use according to any one of claims 1 to 4, wherein said proliferative disease is tumor metastasis or tumor recurrence.

8. A method for identifying non-catalytic cysteine residues in RNA-binding proteins, wherein said method comprises the following steps:
a. Selecting a RNA-binding protein,
b. Recombinant expression and/or purification of the selected RNA-binding protein,
c. Posttranslational chemical mutagenesis of the RNA-binding protein *in vitro* using cysteine-directed probes,
d. Limited proteolysis of the chemically modified RNA-binding protein and analysis of the resulting peptide solution for modified cysteine residues,
e. *In vitro* functional assays to assess the activity of the modified cysteine residues of the RNA-binding protein;
f. Site-directed mutagenesis to produce a mutant version of the RNA-binding protein and assessment of the function *in vitro.*

9. An *in vitro* method for identifying an anti-proliferative compound for use in the prevention or treatment of cancer, tumor metastasis or tumor recurrence in a subject, said method comprising the steps of:
a. Selecting a RNA-binding protein, wherein said RNA-binding protein is a IGF2 mRNA-binding protein;
b. Providing cells of a cancer cell model, wherein the cells of said cancer cell model express the IGF2 mRNA-binding protein;
c. Providing the same cancer cell model as in step b., wherein the gene of the IGF2 mRNA-binding protein has been knocked-out in the cells of said cancer cell model or wherein the cells of said cancer cell model express a mutant, non-functional IGF2 mRNA-binding protein;
d. Contacting the cells of steps b. and c. with a test compound,
e. Measuring at least one of the 3D tumor cell spheroid growth, matrigel invasion and anoikis resistance (self-renewal) of the cells contacted with the test compound according step d.,
f. Selecting a test compound as an anti-proliferative compound for use in the prevention or treatment of cancer, tumor metastasis or tumor recurrence, when said test compound leads in the cells of step b., when compared to the cells of step c., to the inhibition of at least one of 3D tumor cell spheroid growth, matrigel invasion and anoikis resistance.

10. The method according to any one of claims 8 or 9, wherein said RNA-binding protein is IGF2BP1.

11. The method according to any one of claims 8 to 10, wherein said cancer cell model comprises a cell line selected from the group consisting of ES-2, A549, MV3, 786-O, Huh7, PANC1, H1975, BE2C, Kelly, C643 and 8305C.

12. The method according to any one of claims 9 to 11, wherein the cells of step b. endogenously or recombinantly express the RNA-binding protein IGF2BP1.

13. The method according to any one of claims 9 to 11, wherein the cells of step c. express a mutant, non-functional IGF2BP1, which comprises mutations of the non-catalytic cysteine residues C253A and C336A.

## Patentansprüche

1. Antiproliferative Verbindung oder eine pharmazeutische Zusammensetzung, umfassend die antiproliferative Verbindung, zur Verwendung bei der Prävention oder Behandlung von Krebs, Tumormetastasen oder Tumorrezidiven bei einem Subjekt, wobei die antiproliferative Verbindung an mindestens einen nicht-katalytischen Cysteinrest eines IGF2 (Insulin-ähnlicher Wachstumsfaktor 2)-mRNA-bindenden Proteins kovalent bindet und dadurch die proliferative Aktivität dieses IGF2-mRNA-bindenden Proteins hemmt,
**dadurch gekennzeichnet, dass** die antiproliferative Verbindung aus *(E)*-N-(2-((7-Nitrobenzo[c][1,2,5]oxadiazol-4-yl)amino)ethyl)-4-oxo-4-phenylbut-2-enamid und (E)-N-Ethyl-4-oxo-4-phenylbut-2-enamid, einschließlich pharmazeutisch verträglichen Salzes, Solvat oder Polymorph davon, einschließlich aller Tautomere und Stereoisomere davon, ausgewählt ist.

2. Antiproliferative Verbindung oder pharmazeutische Zusammensetzung zur Verwendung nach Anspruch 1, wobei das IGF2-mRNA-bindende Protein (IGF2BP) aus IGF2BP1, IGF2BP2 und IGF2BP3 ausgewählt ist.

3. Antiproliferative Verbindung oder pharmazeutische Zusammensetzung zur Verwendung nach Anspruch 1 oder 2, wobei das RNA-bindende Protein IGF2BP1 ist.

4. Antiproliferative Verbindung oder pharmazeutische Zusammensetzung zur Verwendung nach einem der vorstehenden Ansprüche, wobei die antiproliferative Verbindung an den/die nicht-katalytischen Cysteinrest(e) Cys253 und/oder Cys336 von IGF2BP1 selektiv bindet, wenn es sich auf das Polypeptid bezieht, das die Aminosäuresequenz von SEQ ID NO: 1 aufweist.

5. Antiproliferative Verbindung oder pharmazeutische Zusammensetzung zur Verwendung nach einem der vorstehenden Ansprüche, wobei die proliferative Erkrankung aus malignen Tumoren epithelialen, mesenchymalen, (neuro)-endokrinen oder hämatopoetischen Ursprungs ausgewählt ist.

6. Antiproliferative Verbindung oder pharmazeutische Zusammensetzung zur Verwendung nach einem der vorstehenden Ansprüche, wobei die proliferative Erkrankung Krebs ist und aus der Gruppe ausgewählt ist, die aus malignen Tumoren epithelialer, mesenchymaler oder hämatopoetischer Zellen, Brustkrebs, Prostatakrebs, Bauchspeicheldrüsenkrebs, Nebennierenkrebs, Melanom, Lungenkrebs, Dickdarmkrebs, Leukämie (einem flüssigen oder nicht festen Tumor), Weichteil- und Knochensarkomen, neuroendokrinen Tumoren wie Inselzellkarzinom oder medullärem Karzinom der Schilddrüse, Plattenepithelkarzinomen (insbesondere des Kopfes und Halses), Adenokarzinomen, Gliosarkomen wie Glioblastoma multiforme, Leberkrebs, Nierenkrebs, Blasenkrebs, Magenkrebs, Schilddrüsenkarzinomen follikulären Ursprungs, Neuroblastom, Teratom und Eierstockkarzinom; vorzugsweise Ovarialkarzinom, Lungenadenokarzinom, Melanom, Nierenkarzinom, Leberzellkarzinom, Pankreaskarzinom und kindheitsspezifisches Neuroblastom besteht.

7. Antiproliferative Verbindung oder pharmazeutische Zusammensetzung zur Verwendung nach einem der Ansprüche 1 bis 4, wobei die proliferative Erkrankung eine Tumormetastase oder ein Tumorrezidiv ist.

8. Verfahren zum Identifizieren nichtkatalytischer Cysteinreste in RNA-bindenden Proteinen, wobei das Verfahren die folgenden Schritte umfasst:
a. Auswählen eines RNA-bindenden Proteins,
b. rekombinante Expression und/oder Reinigung des ausgewählten RNA-bindenden Proteins,
c. posttranslationale chemische Mutagenese des RNA-bindenden Proteins *in vitro* unter Verwendung von Cystein-gerichteten Sonden,
d. begrenzte Proteolyse des chemisch modifizierten RNA-bindenden Proteins und Analyse der resultierenden Peptidlösung auf modifizierte Cysteinreste,
e. funktionelle *in* vitro-Tests, um die Aktivität der modifizierten Cysteinreste des RNA-bindenden Proteins zu bewerten;
f. zielgerichtete Mutagenese, um eine mutierte Version des RNA-bindenden Proteins und eine Bewertung der Funktion *in vitro* herzustellen.

9. *In* vitro-Verfahren zum Identifizieren einer antiproliferativen Verbindung zur Verwendung bei der Prävention oder Behandlung von Krebs, Tumormetastasen oder Tumorrezidiven bei einem Subjekt, das Verfahren umfassend die Schritte:
a. Auswählen eines RNA-bindenden Proteins, wobei das RNA-bindende Protein ein IGF2-mRNA-bindendes Protein ist;
b. Bereitstellen von Zellen eines Krebszellmodells, wobei die Zellen des Krebszellmodells das IGF2-mRNA-bindende Protein exprimieren;
c. Bereitstellen desselben Krebszellmodells wie in Schritt b., wobei das Gen des IGF2-mRNA-bindenden Proteins in den Zellen des Krebszellmodells ausgeschaltet wurde oder wobei die Zellen des Krebszellmodells ein mutiertes, nicht-funktionelles IGF2-mRNA-bindendes Protein exprimieren;
d. Inkontaktbringen der Zellen aus den Schritten b. und c. mit einer Testverbindung,
e. Messen von mindestens eines von dem 3D-Tumorzellsphäroidwachstum, der Matrigelinvasion und der Anoikisresistenz (Selbsterneuerung) der gemäß Schritt d. mit der Testverbindung in Kontakt gebrachten Zellen,
f. Auswählen einer Testverbindung als eine antiproliferative Verbindung zur Verwendung bei der Prävention oder Behandlung von Krebs, Tumormetastasen oder Tumorrezidiven, wenn die Testverbindung in den Zellen aus Schritt b. im Vergleich zu den Zellen aus Schritt c. zu der Hemmung von mindestens eines von 3D-Tumorzellsphäroidwachstum, Matrigelinvasion und Anoikisresistenz führt.

10. Verfahren nach einem der Ansprüche 8 oder 9, wobei das RNA-bindende Protein IGF2BP1 ist.

11. Verfahren nach einem der Ansprüche 8 bis 10, wobei das Krebszellmodell eine Zelllinie umfasst, die aus der Gruppe ausgewählt ist, die aus ES-2, A549, MV3, 786-O, Huh7, PANC1, H1975, BE2C, Kelly, C643 und 8305C besteht.

12. Verfahren nach einem der Ansprüche 9 bis 11, wobei die Zellen aus Schritt b. das RNA-bindende Protein IGF2BP1 endogen oder rekombinant exprimieren.

13. Verfahren nach einem der Ansprüche 9 bis 11, wobei die Zellen aus Schritt c. ein mutiertes, nicht-funktionelles IGF2BP1 exprimieren, das Mutationen der nicht-katalytischen Cysteinreste C253A und C336A umfasst.

## Revendications

1. Composé antiprolifératif ou composition pharmaceutique comprenant ledit composé antiprolifératif destiné à être utilisé pour la prévention ou le traitement du cancer, des métastases tumorales ou de la récidive tumorale chez un sujet, dans lequel ledit composé antiprolifératif se lie de manière covalente à au moins un résidu de cystéine non catalytique d'une protéine de liaison à l'ARNm d'IGF2 (facteur de croissance insulinomimétique de type 2) et inhibe ainsi l'activité proliférative de ladite protéine de liaison à l'ARNm d'IGF2, **caractérisé en ce que** ledit composé antiprolifératif est choisi parmi *(E)*-N-(2-((7-nitrobenzo[c][1,2,5]oxadiazol-4-yl)amino)éthyl)-4-oxo-4-phénylbut-2-énamide et (E)-N-éthyl-4-oxo-4-phénylbut-2-énamide, y compris leur sel, solvate ou polymorphe pharmaceutiquement acceptable, y compris tous leurs tautomères et stéréo-isomères.

2. Composé antiprolifératif ou composition pharmaceutique destiné à être utilisé selon la revendication 1, dans lequel ladite protéine de liaison à l'ARNm d'IGF2 (IGF2BP) est choisie parmi IGF2BP1, IGF2BP2 et IGF2BP3.

3. Composé antiprolifératif ou composition pharmaceutique destiné à être utilisé selon la revendication 1 ou 2, dans lequel la protéine de liaison à l'ARN est IGF2BP1.

4. Composé antiprolifératif ou composition pharmaceutique destiné à être utilisé selon l'une quelconque des revendications précédentes, dans lequel ledit composé antiprolifératif se lie sélectivement au ou aux résidus de cystéine non catalytiques Cys253 et/ou Cys336 d'IGF2BP1, lorsqu'il est fait référence au polypeptide qui a la séquence d'acides aminés de SEQ ID NO : 1.

5. Composé antiprolifératif ou composition pharmaceutique destiné à être utilisé selon l'une quelconque des revendications précédentes, dans lequel ladite maladie proliférative est choisie parmi tumeurs malignes d'origine épithéliale, mésenchymateuse, (neuro)endocrine ou hématopoïétique.

6. Composé antiprolifératif ou composition pharmaceutique destiné à être utilisé selon l'une quelconque des revendications précédentes, dans lequel ladite maladie proliférative est un cancer et est choisie dans le groupe constitué de tumeurs malignes des cellules épithéliales, mésenchymateuses ou hémopoïétiques, cancer du sein, cancer de la prostate, cancer du pancréas, cancer des glandes surrénales, mélanome, cancer du poumon, cancer du côlon, leucémie (tumeur liquide ou non solide), sarcomes des tissus mous et sarcomes osseux, tumeurs neuroendocrines telles que carcinome des îlots de Langerhans ou carcinome médullaire de la thyroïde, carcinomes épidermoïdes (en particulier de la tête et du cou), adénocarcinomes, gliosarcomes tels que glioblastome multiforme, cancer du foie, cancer du rein, cancer de la vessie, cancer de l'estomac, carcinomes thyroïdiens d'origine folliculaire, neuroblastome, tératome et carcinome de l'ovaire ; de préférence carcinome de l'ovaire, adénocarcinome pulmonaire, mélanome, carcinome rénal, carcinome hépatocellulaire, carcinome du pancréas et neuroblastome spécifique à l'enfant.

7. Composé antiprolifératif ou composition pharmaceutique destiné à être utilisé selon l'une quelconque des revendications 1 à 4, dans lequel ladite maladie proliférative est une métastase tumorale ou une récidive tumorale.

8. Procédé pour l'identification de résidus de cystéine non catalytiques dans des protéines de liaison à l'ARN, dans lequel ledit procédé comprend les étapes suivantes :
a. sélection d'une protéine de liaison à l'ARN,
b. expression recombinante et/ou purification de la protéine de liaison à l'ARN sélectionnée,
c. mutagenèse chimique post-traductionnelle de la protéine de liaison à l'ARN *in vitro à* l'aide de sondes moléculaires dirigées vers la cystéine,
d. protéolyse limitée de la protéine de liaison à l'ARN modifiée chimiquement et analyse de la solution peptidique résultante pour obtenir des résidus de cystéine modifiés,
e. essais fonctionnels *in vitro* pour évaluer l'activité des résidus de cystéine modifiés de la protéine de liaison à l'ARN ;
f. mutagenèse dirigée pour produire une version mutante de la protéine de liaison à l'ARN et évaluation de la fonction *in vitro.*

9. Procédé *in vitro* pour l'identification d'un composé antiprolifératif destiné à être utilisé dans la prévention ou le traitement du cancer, des métastases tumorales ou de la récidive tumorale chez un sujet, ledit procédé comprenant les étapes consistant à :
a. sélectionner une protéine de liaison à l'ARN, dans lequel ladite protéine de liaison à l'ARN est une protéine de liaison à l'ARNm d'IGF2 ;
b. fournir des cellules d'un modèle de cellule cancéreuse, dans lequel les cellules dudit modèle de cellule cancéreuse expriment la protéine de liaison à l'ARNm d'IGF2 ;
c. fournir le même modèle de cellule cancéreuse qu'à l'étape b., dans lequel le gène de la protéine de liaison à l'ARNm d'IGF2 a été invalidé dans les cellules dudit modèle de cellule cancéreuse ou dans lequel les cellules dudit modèle de cellule cancéreuse expriment une protéine de liaison à l'ARNm d'IGF2 mutante et non fonctionnelle ;
d. mettre en contact les cellules des étapes b. et c. avec un composé de test,
e. mesurer au moins l'une parmi croissance des sphéroïdes de cellules tumorales en 3D, invasion de la matrice Matrigel et résistance à l'anoïkis (autorenouvellement) des cellules mises en contact avec le composé de test à l'étape d.,
f. sélectionner un composé de test comme composé antiprolifératif destiné à être utilisé dans la prévention ou le traitement du cancer, des métastases tumorales ou de la récidive tumorale, lorsque ledit composé de test entraîne dans les cellules de l'étape b., par rapport aux cellules de l'étape c., l'inhibition d'au moins l'une parmi croissance des sphéroïdes de cellules tumorales en 3D, invasion de la matrice Matrigel et résistance à l'anoïkis.

10. Procédé selon l'une quelconque des revendications 8 ou 9, dans lequel ladite protéine de liaison à l'ARN est IGF2BP1.

11. Procédé selon l'une quelconque des revendications 8 à 10, dans lequel ledit modèle de cellule cancéreuse comprend une lignée cellulaire choisie dans le groupe constitué de ES-2, A549, MV3, 786-O, Huh7, PANC1, H1975, BE2C, Kelly, C643 et 8305C.

12. Procédé selon l'une quelconque des revendications 9 à 11, dans lequel les cellules de l'étape b. expriment de manière endogène ou recombinante la protéine de liaison à l'ARN IGF2BP1.

13. Procédé selon l'une quelconque des revendications 9 à 11, dans lequel les cellules de l'étape c. expriment une protéine IGF2BP1 mutante et non fonctionnelle qui comprend des mutations des résidus de cystéine non catalytiques C253A et C336A.
